# EUROPÄISCHE PATENTSCHRIFT

(11) **EP 1 664 089 B1**
(45) Veröffentlichungstag und Bekanntmachung des Hinweises auf die Patenterteilung: **16.11.2011**
(21) Anmeldenummer: 04765144.3
(22) Anmeldetag: 13.09.2004
(51) Int. Cl.: C07K 5/078, C07K 5/065, A61K 38/05, A61P 9/00, A61P 7/02

(54) **BASISCH-SUBSTITUIERTE BENZYLAMINANALOGA ALS INHIBITOREN DES GERINNUNGSFAKTORS XA, IHRE HERSTELLUNG UND VERWENDUNG**
BASE-SUBSTITUTED BENZYLAMINE ANALOGS FOR USE AS COAGULATION FACTOR XA INHIBITORS, THE PRODUCTION AND USE THEREOF
SUBSTANCES ANALOGUES A LA BENZYLAMINE, A BASE SUBSTITUEE, EN TANT QU'INHIBITEURS DU FACTEUR DE COAGULATION XA, LEUR REALISATION ET LEUR UTILISATION

(30) Priorität: 11.09.2003 DE 10342108
(43) Veröffentlichungstag der Anmeldung: 07.06.2006
(60) Teilanmeldung: 10013066.5
(73) Patentinhaber: The Medicines Company (Leipzig) GmbH, 04103 Leipzig (DE)
(72) Erfinder: STÜRZEBECHER, Jörg, 99094 Erfurt (DE); STEINMETZER, Torsten, 07743 Jena (DE); SCHWEINITZ, Andrea, 07749 Jena (DE); STÜRZEBECHER, Anne, 99427 Weimar (DE); DÖNNECKE, Daniel, Victoria BC, V8Z 5L8 (CA)
(74) Vertreter: Bösl, Raphael Konrad
(86) Internationale Anmeldenummer: PCT/EP2004/010225
(87) Internationale Veröffentlichungsnummer: WO 2005/026198

(56) Entgegenhaltungen:
- EP-A- 0 669 317
- EP-A- 0 672 658
- EP-A- 1 364 960
- WO-A-00/14110
- WO-A-00/64470
- WO-A-94/29336
- WO-A-97/23499
- WO-A1-2004/062657
- WO-A2-01/96366
- H. WIKSTRÖM, ET AL.: "Development and validation of a chiral capillary electrophoresis method for melagatran and ximelagatran drug substances" JOURNAL OF SEPARATION SCIENCE, Bd. 25, Nr. 15-17, November 2002 (2002-11), Seiten 1167-1174, XP002314207 WILEY-VCH, WEINHEIM, DE ISSN: 1615-9306
- K. LEE, ET AL.: "Noncovalent tripeptidic thrombin inhibitors incorporating amidrazone, amine and amidine functions at P1" BIOORGANIC & MEDICINAL CHEMISTRY LETTERS, Bd. 12, Nr. 7, 8. April 2002 (2002-04-08), Seiten 1017-1022, XP002314087 ELSEVIER SCIENCE, OXFORD, GB
- D. GUSTAFSSON, ET AL.: "Effects of melagatran, a new low molecular weight thrombin inhibitor, on thrombin and fibrinolytic enzymes" THROMBOSIS AND HAEMOSTASIS, Bd. 79, Nr. 1, Januar 1998 (1998-01), Seiten 110-118, XP002093844 SCHATTAUER, STUTTGART, DE ISSN: 0340-6245
- D. GUSTAFSSON, ET AL.: "The direct thrombin inhibitor melagatran and its oral prodrug H 376/95: intestinal absorption properties, biochemical and pharmacodynamic effects" THROMBOSIS RESEARCH, Bd. 101, Nr. 3, 1. Februar 2001 (2001-02-01), Seiten 171-181, XP001197148 ELSEVIER SCIENCE, OXFORD, GB ISSN: 0049-3848
- S. KUENZEL, ET AL.: "4-Amidinobenzylamine-based inhibitors of urokinase" BIOORGANIC & MEDICINAL CHEMISTRY LETTERS, Bd. 12, Nr. 4, 25. Februar 2002 (2002-02-25), Seiten 644-648, XP002245019 ELSEVIER SCIENCE, OXFORD, GB ISSN: 0960-894X

## Beschreibung

Die Erfindung betrifft neue basisch-substituierte Benzylaminanaloga als Inhibitoren des Gerinnungsfaktors Xa, ihre Herstellung und Verwendung zur Therapie und Prophylaxe von kardiovaskulären Erkrankungen und thromboembolischen Ereignissen.

Die gegenwärtig klinisch eingesetzten Antikoagulantien vom Heparin-Typ bzw. die Vitamin-K-Antagonisten werden nicht allen Anforderungen an ein "ideales" Antithrombotikum gerecht. Deshalb wird mit kleinmolekularen Hemmstoffen der Gerinnungsenzyme, speziell von Thrombin und Faktor Xa (F Xa), nach Alternativen gesucht. Ein besonderer Vorteil von F Xa-Hemmstoffen im Vergleich zu Thrombin-Hemmstoffen könnte die geringere Blutungsneigung sein, die sich bei verschiedenen Tierversuchen gezeigt hat. So wurde bei antithrombotisch effektiven Dosen die Blutungszeit nur minimal beeinflußt (J.M. Herbert et al., J. Pharmacol. Exp. Ther. 276, 1030-1038, 1996; K. Sato et al., Br. J. Pharmacol. 123, 92-96, 1998).

Die ersten nichtpeptidischen Verbindungen mit hoher Affinität für F Xa waren symmetrische Bis-benzamidine (Kᵢ = 13 nM für die wirksamste Verbindung BABCH) (J. Stürzebecher et al., Thromb. Res. 54, 245-252 1989). Auch das Naphthamidin-Derivat DX-9065a besitzt zwei basische Gruppen und hemmt F Xa selektiv mit einem Kᵢ = 24 nM (T. Hara et al., Thromb. Haemost. 71, 314-319, 1994). Der mit DX-9065a strukturell verwandte Inhibitor YM-60828 (K. Sato et al. Eur. J. Pharmacol. 339, 141-146, 1997) ist noch wirksamer (Kᵢ = 1.3 nM). Inzwischen wurde eine ganze Reihe weiterer bis-basischer Verbindungen beschrieben, bei denen z. B. zwei Benzamidin-Reste über einen Oxazolin-Ring (Kᵢ = 18 nM) (M.L. Quan et al., Bioorg. Med. Chem. Lett. 7, 2813-2818, 1997) bzw. eine Carboxymethylalkyl-Kette (Ki = 34 nM) verknüpft sind (T.P. Maduskuie et al., J. Med. Chem. 41, 53-62, 1998). Nachteil der bis-basischen Verbindungen ist insbesondere die geringe Bioverfügbarkeit nach oraler Gabe.

Auch Hemmstoffe für F Xa, die nur eine basische Gruppe enthalten, wurden beschrieben. N-substituierte Amidino-phenoxypyridine (Kᵢ = 0,11 nM für BX-807834) wurden auf der Basis von BABCH entwickelt (R. Mohan et al., Bioorg. Med. Chem. Lett. 8, 1877-1882, 1998; G.B. Phillips et al. J. Med. Chem. 41, 3557-3562, 1998). Amide des Nα-Adamantyloxycarbonyl-3-amidinophenylalanins (Kᵢ = 74 nM für die wirksamste Verbindung) sind selektive Hemmstoffe des F Xa (S. Sperl et al., Biol. Chem. 381, 321-329, 2000), während Nα-arylsulfonyl-aminoacylierte Ester des 3-Amidinophenylalanins eine geringe Hemmwirkung (Kᵢ = 840 nM für TAPAM) besitzen (J. Stürzebecher et al., Thromb. Res. 54, 245-252, 1998). Die WO 96/10022 offenbart Hemmstoffe, die überhaupt keine starke Ladung mehr besitzen (Kᵢ = 3,0 nM für die wirksamste Verbindung). Eine weitere Serie von wirksamen Faktor Xa-Hemmstoffen ohne basische Substituenten wurden kürzlich von Choi-Sledeski et al. (J. Med. Chem. 46, 681-684, 2003) beschrieben.

Bisher wurden nur wenige Peptide als Hemmstoffe für F Xa beschrieben, die sich von der Substrat-Sequenz Ile-Glu-Gly-Arg ableiten. Die von Kettner und Shaw (Thromb. Res. 22, 645-652, 1981) beschriebenen Chlormethylketone hemmen F Xa irreversibel und sind nicht für in vivo-Anwendungen geeignet. Dagegen sind die Peptide SEL 2489 (Kᵢ = 25 nM) und SEL 2711 (Kᵢ = 3 nM) außerordentlich wirksam (J. A. Ostrem et al., Biochemistry 37, 1053-1059,1998). Auch einige Peptidyl-Arginin-Aldehyde und Peptidyl-Arginyl-Ketone wurden beschrieben, die neben Argininal oder einem Arginyl-Ketonderivat, wie z.B. Arginyl-Ketothiazol in P3-Position ein D-Arginin bzw. eine unnatürliche basische Aminosäure, wie z.B. 4-Amidinophenylalanin, 3- oder 4-Amidinopiperidinylalanin und 4-Guanidinophenylalanin in P3 besitzen (Z. H. Jonathan, Bioorg. Med. Lett. 9, 3459-3464, 1999 und Übersichtsarbeit: Zhu und Scarborough Current Opinion in Cardiovascular, Pulmonary & Renal Investigational Drugs 1999, 1, 63-88).) In der Anmeldung WO 01/96366 sind Hemmstoffe offenbart, die sich von acyliertem Amidinobenzylamin ableiten und neben einer natürlichen Aminosäure in P2 einen D-Ser-Ether oder ein vergleichbares Derivat einer unnatürlichen Aminosäure enthalten. Verbindungen diese Typs hemmen sowohl F Xa (Kᵢ = 30 nM für die wirksamste Verbindung) als auch die Gerinnung von menschlichem Blutplasma sehr wirksam. Allerdings haben Verbindungen diese Typs nur unzureichende pharmakokinetische Eigenschaflen für eine Anwendung in vivo; sie werden nach oraler Gabe kaum resorbiert und im Versuchstier nach i.v.-Gabe sehr schnell aus der Zirkulation eliminiert.

In dem U.S. Patent Nr. US 5,914,319 wurden Thrombinhemmstoffe beschrieben, die in P3-Position ein d-Homophenylalanin oder d-Homocyclohexylalanin besitzen und auch eine schwache Faktor Xa-Inhibierung mit Hemmkonsten im mikromaolaren Bereich zeigen (für Faktor Xa: Kₐₛₛ < 5,5 x 10⁶ l/mol, entspricht ca. Kᵢ > 0,18 µM). Diese Inhibitoren besitzen jedoch zwingend eine Aminosäure in P2-Position, also Analoga des Prolins oder N(Alkyl)Glycin-Derivate. Auch ist die Thrombinaffinität deutlich erhöht und das Selektivitätsverhältnis (Kᵢ für Thrombin/Kᵢ für F Xa) ist für die angegebenen Verbindungen < 0,08.

Dipeptide, welche Inhibitoren von Thrombin und Faktor Xa sind, werden von Künzel et al. offenbart ("4-Amidinobenzylamine-Based Inhibitors of Urokinase" in Bioorganic & Medicinal Chemistry Letters, 2002, 12(4), 644-648). Diese Dipeptide unterscheiden sich jedoch von denen der vorliegenden Erfindung darin, dass sie anstelle des Ring-enthaltenden Aminosäurerests einen Serin-Rest enthalten.

Ferner beschreibt WO 2004/062657 A1 die Verwendung von acyliertem 4-Amidino- oder 4-Guanidinobenzylamin zur Inhibierung von Plasmakallikrein (PK), Faktor XIa und Fator XIIa, insbesondere zur Verhinderung der Gerinnungsaktivierung an künstlichen Oberflächen und zur systemischen Gabe als Antikoagulanzien/Antithrombotika. Die in dieser Schrift vorgestellten Verbindungen unterscheiden sich jedoch strukturell von denen der vorliegenden Erfindung.

Der Erfindung liegt daher die Aufgabe zu Grunde, einen für therapeutische Anwendungen geeigneten Wirkstoff anzugeben, der den Gerinnungsfaktor Xa mit hoher Aktivität und Spezifität hemmt und der vorzugsweise nach i.v.-, s.c.- oder oraler Gabe möglichst lange im Körper zirkuliert.

Überraschend wurde gefunden, dass acyliertes Amidinobenzylamin gemäß der im Patentanspruch 1 angeführte allgemeinen Formel I, 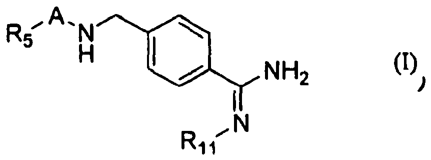 wobei
A P₂-P₁ ist, mit 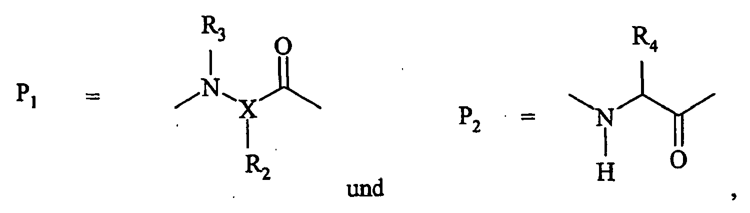 insbesondere Verbindungen des 4-Amidinobenzylamins, sowohl Faktor Xa sehr wirksam inaktivieren als auch langsam aus der Zirkulation eliminiert werden, wenn neben der Amidinofunktion weitere geladene oder polare Gruppen eingeführt werden, wobei es sich vor allem herausgestellt hat, dass D-Homophenylalanin, D-Homotyrosin bzw. D-Homo-4-pyridylalanin und dessen Derivate an der Position P₂ gemäß der allgemeinen Formel I besonders wirksam sind. Durch die Verwendung von ausgewählten α-Aminosäuren in P2-Position konnte auch die Selektivität als Faktor Xa-Hemmstoffe entscheidend erhöht werden; was besonders überraschend war.

Zur klarstellung wird darauf hingewiesen, dass die Benennung der Reste P₂ und P₁ in dem Struktursegment A der allgemeinen Formel I sich nicht auf die sonst üblicherweise verwendete Nomenklatur der Aminosäurereste in Peptidsubstraten von Serinproteasen und davon abgeleiteten Inhibitoren bezieht, wie sie von Schechter und Berger eingeführt wurde (Schechter und Berger, Biochem. Biophys. Res. Comm. 27, 157-162 (1967)). In allen Teilen der Erfindung, d.h. sowohl in der Beschreibung als auch in den Ansprüchen gelten die folgenden Definitionen:

Der Buchstabe P in Zusammenhang mit einer Zahl von 1 bis 3 in normaler Schrift, d.h. P1, P2 oder P3, wird für Aminosäurereste und deren Derivate entsprechend der Nomenklatur von Schechter und Berger verwendet. Dagegen steht der Buchstabe P in Zusammenhang mit einer tiefgestellten 1 oder 2, d.h. P₁ oder P₂, für Aminosäurereste und deren Derivate als Bestandteile der Struktur A in Formel I der vorliegenden Erfindung. Dabei entspricht substituierte oder unsubstituierte natürliche oder unatürliche Aminosäure P₁ in der Struktur A P2 nach Schechter und Berger und die in der D-Konfiguration vorliegende substituierte oder unsubstituierte natürliche oder unatürliche Aminosäure P₂ in der Struktur A entspricht P3 nach Schechter und Berger.

Ein Gegenstand der vorliegenden Erfindung ist daher eine Verbindung der allgemeinen Formel I 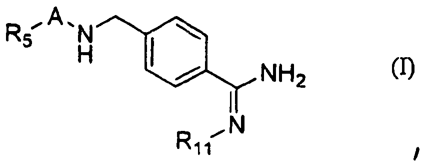 wobei A P₂-P₁ ist, mit 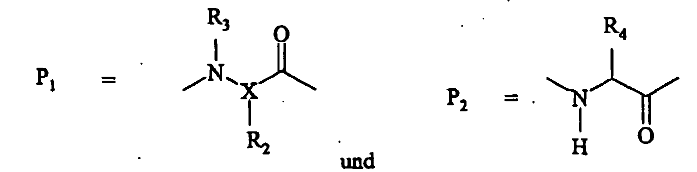 ist;
- X: ein N oder CH, vorzugsweise an CH; R₂ ein H, -CH₂-OR₇ oder -CH₂-OCOOR₇ ist, wobei R₇ ein H oder ein verzweigter oder unverzweigter Alkylrest mit 1-5, insbesondere 1-3 C-Atomen, ist, oder R₂ ist ein -CH₂-CH₂-COOR_{7*}, wobei R_{7*} ein H oder ein verzweigter oder unverzweigter Alkylrest mit 1-5 C-Atomen, vorzugsweise Ethyl, ist;
- R₃: ein H ist;
- R₄: -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Aryl- oder Heteroarylrest ist, wobei der Aryl- oder Heteroarylrest vorzugsweise 5 bis 14, insbesondere 5 bis 6 C-Atome im Ring und im Falle des Heteroarylrestes vorzugsweise 1 bis 3 N als Heteroatome besitzt, und wobei P₂ in der Struktur A der allgemeinen Formel I in der D- oder L-Konfiguration, vorzugsweise in der D-Konfiguration vorliegt;
- R₅: -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R_{9*}, -SO₂-NH-R_{9*}, wobei R_{9*} ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂- OCOOR₇, mit R₇ wie oben definiert, -NH₂, -NO₂, -COOR₁₀, -CH₂-COOR₁₀-Gruppe oder ein Cl-, F- oder Br-Atom ist und wobei R₁₀ ein H oder ein Alkylrest mit 1 bis 6, insbesondere mit 1 bis 4 C-Atomen, vor allem Ethyl, ist;
- R₁₁: H, OH, oder -COOR₁₂ ist, wobei R₁₂ ein verzweigter oder unverzweigter Alkylrest mit 1 bis 8, vorzugsweise 1 bis 6 C-Atomen ist;
oder eine Verbindung der allgemeinen Formel I in Form ihres Salzes;
mit Ausnahme folgender Verbindungen:
Benzylsulfonyl-D-hPhe-Ser-4-Amidinobenzylamid,
Benzylsulfonyl-D-hAla(4-Pyridyl)-Ser-4-Amidinobenzylamid,
Benzylsulfonyl-D-hTyr-Ser-4-Amidinobenzylamid,
4-NH₂-Benzylsulfonyl-D-hPhe-Gly-4-Amidinobenzylamid,
Benzylsulfonyl-D-Indanylglycyl-Gly-4-Amidinobenzylamid,
Benzylsulfonyl-D/L-hAla(4-Pyridyl)-Gly-4-Amidinobenzylamid und
4-Pyridylmethylsulfonyl-D-hPhe-Gly-4-Amidinobenzylamid:,
wobei der C-terminale aromatische Benzamidinrest an 1, 2 oder 3 Positionen zusätzlich mit einem Halogen, insbesondere Fluor oder Chlor, oder einem Methyl-, Ethyl-, Propyl-, Methoxy-, Ethoxy-, oder Propoxyrest subsituiert sein kann.

Sofern nichts anderes definiert ist, bedeutet der Begriff "Substituent" bzw. "substituiert" gemäß der vorliegenden Erfindung beispierweise -OH, -NH₂, -NO₂, -COOH, - COOCH₂CH₃ oder ein Halogen, wobei der Begriff "Halogen" im allgemeinen Fluor, Chlor oder Brom, vorteilhafterweise Fluor oder Chlor, bedeutet.

Weiterhin bevorzugt ist daher eine Verbindung gemäß allgemeiner Formel I, dadurch gekennzeichnet, dass der Substituent -OH, -NH₂, -NO₂, -COOH, -COOCH₂CH₃, Fluor, Chlor oder Brom, insbesondere Fluor oder Chlor, ist.

Als Alkylrest wird im allgemeinen, sofern nicht anders definiert, ein Rest mit vorteilhafterweise 1-5 C-Atomen, beispielweise Ethyl, und als Cycloalkyl-, Aryl-, Aralkylrest wird im allgemeinen, sofern nicht anders definiert, ein Rest mit vorteilhafterweise 4 bis 14, beispielweise 6 bis 10, wie 6 C-Atomen als Ringatome bezeichnet. Der Begriff "Hetero" bedeutet im allgemeinen, sofern nicht anders definiert, beispeilsweise N, S oder O, vorteilhafterweise insbesondere N, wobei bei einem Heteroarylrest mindestens ein C-Atom des Ringes durch ein Heteroatom ersetzt ist, zum Beispiel sind 1, 2 oder 3 C-Atome des Ringes insbesondere durch N ersetzt.

Eine weitere Ausführungsform der vorliegenden Erfindung betrifft eine Verbindung gemäß allgemeiner Formel I, dadurch gekennzeichnet, dass die Verbindungen als Salze vorliegen, vorzugsweise mit Mineralsäuren oder mit geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.

Noch ein weiterer Gegenstand der vorliegenden Erfindung ist ein Verfahren zur Herstellung einer Verbindung gemäß allgemeiner Formel 1, dadurch gekennzeichnet, dass sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird.

Ferner ist ein Gegenstand der vorliegenden Erfindung ein Arzneimittel enthaltend eine Verbindung gemäß allgemeiner Formel 1 sowie pharmazeutisch geeignete Hilfs- und/oder Zusatzstoffe.

Bevorzugt ist ein entsprechendes Arzneimittel, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Emulsion oder Suspension, eines Globuli, Styli, Aerosols, Puders, einer Paste, Creme oder Salbe eingesetzt wird.

Auch Gegenstand der vorliegenden Erfindung ist die Verwendung einer Verbindung gemäß allgemeiner Formel 1 zur Herstellung eines Arzneimittels als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.

Im Detail sind repräsentative Verbindungen gemäß der vorliegenden Erfindung Verbindungen gemäß den Verbindungen 11 bis 20 und 22 bis 65 der Tabelle 1.

Unter den einzelnen besonders vorteilhaften Verbindungen gehören jedoch auch Verbindungen, bei denen in den genannten Strukturen der Glycinrest mit dem Strukturelement 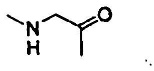 jeweils ersetzt ist durch einen Serinrest mit dem Strukturelement 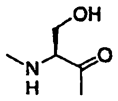 oder durch einen Glutaminsäurerest mit dem Strukturelement 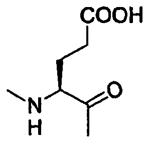 oder durch einen Glutamin-γ-ethylester mit dem Strukturelement

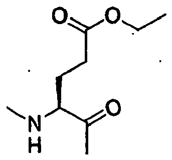

Beispielsweise sind dies folgende Strukturen mit einem Serinrest:

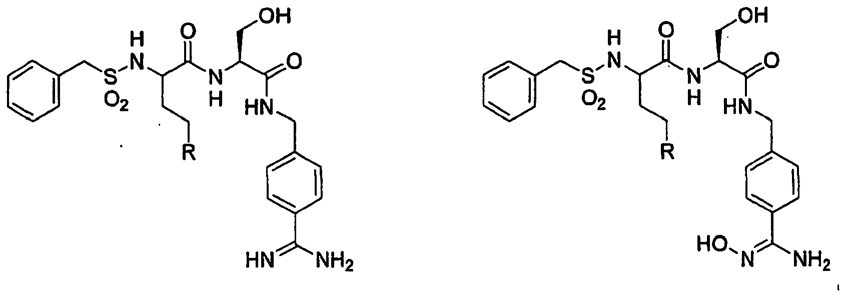

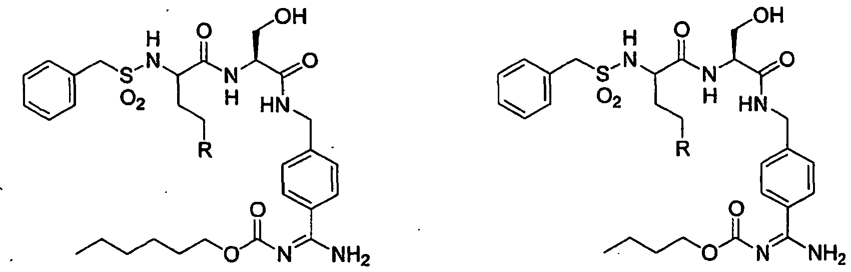

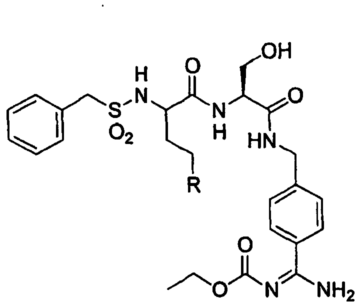

Weitere geeignete Verbindungen sind in folgenden beschtrieben:

Eberfalls geeignet sind Verbindungen wie vorstehend genannt mit der Ausnahme, dass R₂ -(CH₂)ₐCONHR_{7*} oder -(CH₂)ₐCONHR_{7**} mit a = 1, 2, oder 3 ist, wobei R_{7**} ein Arylrest, beispielweise ein Phenylrest oder ein Aralkyl, vorzugsweise ein Benzoylrest ist oder ein Heteroarylrest mit ein bis zwei N-, S- oder O-Heteroatomen, vorteilhafterweise N-Heteroatomen ist.

Ein weiterere Gruppe der geeigneten Verbindungen sind Verbindungen wie vorstehend genannt, wobei R₂-(CH₂)ₐCONHR_{7*} oder -(CH₂)ₐCONHR_{7**} mit a = 1, 2, oder 3 ist und wobei R_{7**} substituiert ist mit mindestens einem Halogen, einer Methyl-, einer Ethyl-, einer Amino-, einer Hydroxy-, einer Vitro-, einer -COOH, einer -CH₂COOH, oder einer - CH₂NH₂- Gruppe.

Darüber hinaus geeignete Verbingen sind Verbindungen wie vorstehend genannt, wobei R₂ ein -(CH₂)ₙ-NH₂ ist, mit n= 1, 2, 3, 4 oder 5, vorteilhafterweise 1 oder 4, ist.

Auch geeignet sind Verbindungen wie vorstehend genannt, wobei R₅ R₉ = H ist.

Weitere reprösentative geeignete Verbindungen sind soldie Verbindungen wie vorstehend genannt, wobei R₄ eine -CH₂-SR₈ oder -CH₂CH₂-SR₈ Gruppe ist. Beispiele derartiger Verbindungen sind solche, in denen R₅ eine -SO₂R_{9*} oder eine -SO₂CH₂R_{9*} Gruppe ist oder in denen R₅ eine -SO₂R_{9*} oder eine -SO₂CH₂R_{9*} Gruppe ist ist oder in denen R₅ eine -SO₂R_{9*} oder eine -SO₂CH₂R_{9*} Gruppe ist.

Ebenso sind Verbindungen geeignet, in denen R₅ eine -SO₂R_{9*} oder eine -SO₂CH₂R_{9*} Gruppe oder in denen R₅ eine -SO₂R_{9*} oder eine -SO₂CH₂R_{9*} Gruppe ist oder in denen R₅ eine -SO₂R_{9*} oder eine -SO₂CH₂R_{9*} Gruppe ist und in denen R_{9*} ein Phenylrest, ein Cyclohexylrest, ein Pyridylrest oder ein Pyridyl-N-oxidrest ist

Ebenso geeignete solche in denen R₅ eine -SO₂R_{9*} oder eine -SO₂CH₂R_{9*} Gruppe ist oder in denen R₅ eine -SO₂R_{9*} oder -SO₂CH₂R_{9*} Gruppe ist oder in denen R₅ eine -SO₂R_{9*} oder -SO₂CH₂R_{9*} Gruppe ist und in denen R_{9*} ein substituierter Phenyl- oder Cyclohexyl- oder Pyridyl- oder Pyridyl-N-oxidrest ist, wobei der Substituent eine -OH, -O-COOR₇, - CH₂OCOOR₇, mit R₇ wie oben definiert, NH₂-, NO₂-, -COOR₁₀, -CH₂COOR₁₀- Gruppe oder ein Cl- oder F- oder Br-Atom sein kann.

Ebenso sind darüber hinaus Verbindungen geeignet in denen R₅ eine -SO₂R_{9*} oder -SO₂CH₂R_{9*} Gruppe ist oder in denen R₅ eine -SO₂R_{9*} oder -SO₂CH₂R_{9*} Gruppe ist oder in denen R₅ eine -SO₂R_{9*} oder -SO₂CH₂R_{9*} Gruppe ist und in denen R_{9*} ein substituierter Phenyl- oder Cyclohexyl- oder Pyridyl- oder Pyridyl-N-oxidrest ist, wobei der Substituent eine -OH, - O-COOR₇, -CH₂OCOOR₇, mit R₇ wie oben definiert, NH₂-, NO₂-, -COOR₁₀, - CH₂COOR₁₀- Gruppe oder ein Cl- oder F- oder Br-Atom sein kann und in denen R₁ - (CH₂)ₐCONHR₆ oder -(CH₂)ₐCONHR_{6*} mit a = 0, 1, 2, 3, 4 oder 5, vorzugsweise 0, 1 oder 2 ist, wobei R_{6*} ein Arylrest, vorteilhafterweise ein Phenylrest ist.

Ebenso sind auch Verbindungen geeignet in denen R₅ eine -SO₂R_{9*} oder eine -SO₂CH₂R_{9*} Gruppe ist oder in denen R₅ eine -SO₂R_{9*} oder -SO₂CH₂R_{9*} Gruppe ist oder in denen R₅ eine -SO₂R_{9*} oder eine -SO₂CH₂R_{9*} Gruppe ist und in denen R_{9*} ein substituierter Phenyl- oder Cyclohexyl- oder Pyridyl- oder Pyridyl-N-oxidrest ist, wobei der Substituent eine -OH,-O-COOR₇, -CH₂OCOOR₇, mit R₇ wie oben definiert, eine NH₂-, NO₂-, -COOR₁₀,-CH₂COOR₁₀- Gruppe oder ein Cl- oder F- oder Br-Atom sein kann und in denen R₂ -CH₂-CH₂-CONHR₇∗ oder -CH₂CH₂CONHR₇∗∗ oder -CH₂CH₂COOR₇∗∗ ist wobei R₇∗∗ ein Arylrest, -beispielweise ein Benzyl- oder Phenylrest ist.

Auch sind Verbindungen geeignet in denen R₅ eine -SO₂R₉∗ oder eine -SO₂CH₂R₉∗ Gruppe ist oder in denen R₅ eine -SO₂R₉∗ oder eine -SO₂CH₂R₉∗ Gruppe und oder in denen R5 eine -SO₂R₉∗ oder -SO₂CH₂R₉∗ Gruppe ist und in denen R₉∗ ein substituierter Phenyl- oder Cyclohexyl- oder Pyridylrest ist, wobei der Substituent eine -OH, -O-COOR₇, -CH₂OCOOR₇, mit R₇ wie oben definiert, eine NH₂-, NO₂-, -COOR₁₀, -CH₂COOR₁₀- Gruppe oder ein Cl- oder F- oder Br-Atom sein kann und in denen R₄ eine -CH₂-SR₈ oder -CH₂CH₂-SR₈ -Gruppe ist.

Ebenso sind Verbindungen vorteilhaft wie vorstehend genannt, wobei R₈ ein Pyridyl-N-oxid- Rest ist.

Außerdern sind Verbindungen, vorteilhaftin denen P₁ ein Prolylrest oder ein Azetidincarbonsäurerest ist.

Ebenso sind Verbindungen vorteilhaft in denen P₂ ein 4-N-oxid-Pyridylhomoalaninrest ist. Auch sind Verbindungen vorteilhaft in denen P₂ ein Lysyl- oder ein a,β-Diaminopropionsäurerest ist.

Daher ist ferner auch Gegenstand der vorliegenden Erfindung eine Verbindung der allgemeinen Formel I 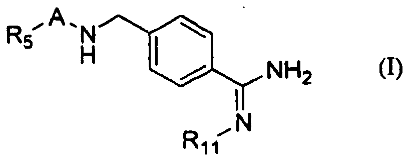 wobei
A P₂- P₁ ist, mit
- P1 =: 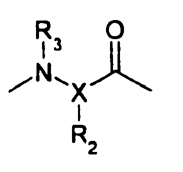 und
- P2 =: 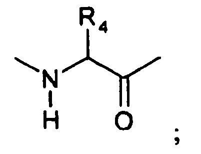
- X =: N oder CH, vorzugsweise CH;
- R₂: eine -(CH₂)ₐCONHR₇∗ oder -(CH₂)ₐCONHR₇∗∗ Gruppe ist, wobei a = 1, 2 oder 3 und
- R₇∗: ein H oder ein verzweigter oder unverzweigter Alkylrest mit 1-5 C-Atomen, vorzugsweise Ethyl;
- R₇∗∗: ein Arylrest oder ein Heteroarylrest mit 1 -2 Heteroatomen ausgewählt aus N, S oder O ist;
- R₃: ein H ist;
- R₄: -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Aryl- oder Heteroarylrest ist, wobei der Aryl- oder Heteroarylrest 5 bis 14, insbesondere 5 bis 6 C-Atome im Ring und im Falle des Heteroarylrestes vorzugsweise 1 bis 3 N als Heteroatome besitzt, und wobei P₂ in der Struktur A der allgemeinen Formel I in der D-oder L-Konfiguration, vorzugsweise in der D-Konfiguration vorliegt;
- R₅: -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R₉∗, -SO₂-NH-R₉∗, wobei R₉∗ ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₇, mit R₇ wie oben definiert, -NH₂, -NO₂, -COOR₁₀, -CH₂-COOR₁₀-Gruppe oder ein Cl-, F- oder Br-Atom ist und wobei R₁₀ ein H oder ein Alkylrest mit 1 bis 6, insbesondere mit 1 bis 4 C-Atomen, vor allem Ethyl, ist;
- R₁₁,: H, OH, oder -COOR₁₂ ist, wobei R₁₂ ein verzweigter oder unverzweigter Alkylrest mit 1 bis 8, vorzugsweise 1 bis 6 C-Atomen ist;
oder eine Verbindung der allgemeinen Formel I in Form ihres Salzes.

Bevorzugt ist eine Verbindung gemäß der allgemeinen Formel I, wobei in R₅ R₉ = H ist.

Weiterhin bevorzugt ist eine Verbindung nach der allgemeinen Formel I, wobei R₄ eine -CH₂-SR₈ oder -CH₂CH₂-SR₈ Gruppe ist.

Besonders bevorzugte Verbindungen sind Verbindungen nach der allgemeinen Formel I, wobei mindestens eine Carboxylgruppe als Ester, bevorzugt als Ethylester, geschützt vorliegt.

Ganz allgemein ist ein Prodrug ein pharmazeutisch inaktives Derivat der entsprechenden pharmazeutisch wirksamen Substanz, das nach oraler Gabe spontan oder enzymatisch unter Freisetzung der pharmazeutisch wirksamen Substanz biotransformiert wird. Im Sinne eines Prodrugs wird eine als Ester, zum Beispiel als Ethylester, geschützte Carboxylgruppe in einer Verbindung nach der allgemeinen Formel I erst nach Flufnahme im Körper in eine Carboxylgruppe umgewandelt.

Folglich versteht man als Prodrug beispielsweise Verbindungen der allgemeinen Formel I, bei denen zusätzlich oder ausschließlich ein oder mehrere Carboxylgruppen in Form ihrer Alkylester mit einem verzweigten oder unverzweigten Alkyl mit 1-5 C-Atomen, vorzugsweise Ethyl, und/oder bei denen ein oder mehrere Hydroxylgruppen als Carbonate, bei denen der endständige Rest gleich R₇, wie oben definiert, vorliegen können. Ein Prodrug im Sinne der vorliegenden Erfindung ist beispielsweise auch ein Amidino- oder Guanidinobenzylaminderivat gemäß der allgemeinen Formel I, bei dem der Amidino- bzw. Guanidinobenzylaminrest als Hydroxyamidin bzw. Hydroxyguanidin oder als Alkyloxycarbonylderivat mit vorzugsweise einem verzweigten oder unverzweigten Alkylrest mit 1-5 C-Atomen, vorzugsweise Ethyl, vorliegt.

Ebenso bevorzugte Verbindungen sind Verbindungen nach der allgemeinen Formel I in Form eines Prodrugs, bei dem ein oder mehrere Carboxylgruppen in Form ihrer Alkylester mit einem verzweigten oder unverzweigten Alkyl mit 1-5 C-Atomen, vorzugsweise Ethyl, und/oder bei dem ein oder mehrere Hydroxylgruppen als Carbonate, bei denen der endständige Rest gleich R₇, wie oben definiert vorliegen, und/oder bei dem der Amidino- bzw. Guanidinobenzylaminrest als Hydroxyamidin bzw. Hydroxyguanidin oder als Alkyloxycarbonylderivat vorliegt.

Beispielhaft sind dies Verbindungen, bei denen
- R₂: ein H, -CH₂-CH₂-COOH, -CH₂-CH₂-COOCH₂CH₃ oder -CH₂OH ist;
- R₃: ein H ist;
- R₄: ein -(CH₂)₂-R₈, -CH₂NH₈, -(CH₂)₂NHR₈ oder ein -CH₂-4-Hydroxycyclohexyl-Rest ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
- R₅: ein Methylsulfonyl, Ethylsulfonyl, n-Propylsulfonyl, iso-Propylsulfonyl, Butylsulfonyl, Benzylsulfonyl, n-Butylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylsulfonyl, N-(oxid)-pyridylmethylsulfonyl, -CH₂-COOH oder ein -CH₂COOCH₂CH₃ Rest ist;
wobei R₁₁ H, OH oder -COOR₁₂ mit R₁₂ ein verzweigter oder unverzweigter Alkylrest mit 2, 4 oder 6 C-Atomen ist.

Ein weiterer Gegenstand der vorliegenden Erfindung ist daher eine Verbindung der allgemeinen Formel I 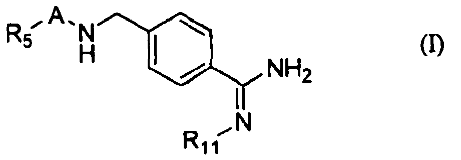 wobei
A P₂-P₁ ist, mit
- P₁=: 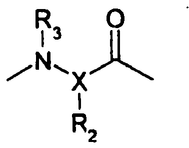 und
- P2 =: 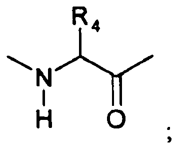
- R₂: ein H, -CH₂-CH₂-COOH, -CH₂-CH₂-COOCH₂CH₃ oder -CH₂OH ist;
- R₃: ein H ist;
- R₄: ein -(CH₂)₂-R₈, -CH₂NHR₈, -(CH₂)₂NHR₈ oder ein -CH₂-4-Hydroxycyclohexylrest ist, wobei R₈ ein einfach oder mehrfach substituierter oder unsubstituierter Aryl- oder Heteroarylrest ist, wobei der Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
- R₅: ein Methylsulfonyl, Ethylsulfonyl, n-Propylsulfonyl, iso-Propylsulfonyl, Butylsulfonyl, n-Butylsulfonyl, Benzylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylensulfonyl, N-(oxid)-pyridylmethylensulfonyl, -CH₂-COOH oder ein -CH₂COOCH₂CH₃ Rest ist;
- X: CH ist;
- R₁₁: H, OH oder -COOR₁₂ mit R₁₂ ein verzweigter oder unverzweigter Alkylrest mit 2, 4 oder 6 C-Atomen ist,
oder eine Verbindung der allgemeinen Formel I in Form ihres Salzes;
mit Ausnahme folgender Verbindungen:
Benzylsulfonyl-D-hPhe-Ser-4-Amidinobenzylamid,
Benzylsulfonyl-D-hAla(4-Pyridyl)-Ser-4-Amidinobenzylamid,
Benzylsulfonyl-D-hTyr-Ser-4-Amidinobenzylamid,
4-NH₂-Benzylsulfonyl-D-hPhe-Gly-4-Amidinobenzylamid,
Benzylsulfonyl-D-Indanylglycyl-Gly-4-Amidinobenzylamid,
Benzylsulfonyl-D/L-hAla(4-Pyridyl)-Gly-4-Amidinobenzylamid und
4-Pyridylmethylsulfonyl-D-hPhe-Gly-4-Amidinobenzylamid.

Andere bevorzugte Verbindungen sind Verbindungen des allgemeinen Formel I, bei denen R₄ ein -CH₂-CH₂-R₈ Rest ist, wobei R₈ ein Aryl- oder Heteroarylrest mit 4-6 Ringatomen ist, der 1 oder 2 Heteroatome, vorzugsweise N, besitzt und der mit einer oder mehreren -NH₂ und/oder-OH Gruppen substituiert ist, vorzugsweise ist P₂ in der Struktur A der allgemeinen Formel I von einem Homophenylalanin, Homotyrosin, Indanylglycin oder 4-Pyridylhomoalanin abgeleitet, und die P₂- Aminosäure liegt insbesondere in der D-Konfiguration vor.

Ebenso sind die Verbindungen eine oder mehr oder alle der Verbindungen 22 bis 64 der Tabelle 1 Gegenstand der Erfindung.

Neben der Inaktivierung von Faktor Xa werden die zusätzlich geladenen 4-Amidinobenzylamin-Derivate gemäß der vorliegenden Erfindung wie bereits erwähnt auf vorteilhafte und überraschende Weise sehr langsam eliminiert, so dass die erfindungsgemäßen Verbindungen eine neue Gruppe von hochaktiven F Xa-Hemmstoffen darstellen.

Die Verbindungen liegen in der Regel als Salze, vorzugsweise mit Mineralsäuren oder geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.

Die Verbindungen der allgemeinen Formel I können in prinzipiell bekannter Weise, wie nachfolgend beschrieben, beispielsweise wie folgt hergestellt werden, wobei im allegemeinen sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird:

Aus dem kommerziell erhältlichen 4-Cyanobenzylamin (Showa Denko, Japan) wird das Boc-geschützte 4-Acetyloxamidinobenzylamin nach dem Fachmann bekannten Verfahren gewonnen. Nach Abspaltung der Boc-Schutzgruppe erfolgt die Ankopplung der weiteren Aminosäuren und der Schutzgruppe R₅ mittels Standardkopplungsmethoden mit Boc als N-terminaler Schutzgruppe. Die zweite Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidinobenzylamin, aufgebaut. Die meisten Zwischenprodukte kristallisieren gut und lassen sich damit einfach reinigen. Die Endreinigung der Hemmstoffe erfolgt auf der letzten Stufe vorzugsweise über präparative, reversed-phase HPLC.

Ein weiterer Gegenstand der Erfindung ist daher ein Verfahren zur Herstellung einer Verbindung nach der allgemeinen Formel 1, wobei sequentiell die entsprechenden Aminosäuren an ein an der Amidinogruppe geschütztes Amidinobenzylamin. angekoppelt werden, wobei die N-terminale Aminosäure entweder den R₅-Rest bereits trägt oder dieser anschließend daran gebunden wird.

Beispielsweise kann sequentiell die entsprechende Aminosäure an ein 4-Acetyloxomidinebenzylamin odes an ein 4-(Benzyloxycarbonylamidino) benzylamin angekoppelt werden.

Ein anderer Gegenstand der Erfindung ist auch ein Arzneimittel enthaltend eine erfindungsgemäße Verbindung sowie weitere pharmazeutisch geeignete Hilfs- und/oder Zusatzstoffe. Geeignete Hilfs- und/oder Zusatzstoffe, die z.B. der Stabilisierung und/oder konservierung des Arzneimittels dienen, sind dem Fachmann allgemein geläufig (z.B. Sucker H. et al., (1991) Pharmazeutische Technologie, 2. Auflage, Georg Thieme Verlag, Stuttgart). Hierzu zählen beilspielsweise physiologische Kochsalzlösungen, Ringer-Dextrose, Ringer-Laktat, entmineralisiertes Wasser, Stabilisatoren, Antioxidantien, komplexbildner, antimikrobielle Verbindungen, Proteinaseinhibitoren und/oder inerte Gase.

Das Arzneimittel könnte beispielsweise in parenteraler Anwendungsform insbesondere in intraartieller, intravenöser, intramuskulärer oder subcutaner Form, in enteraler Anwendungsform, insbesondere zur oralen oder rektalen Anwendung, oder in topischer Anwendungsform, insbesondere als Dermatikum, angewendet werden. Bevorzugt sind intravenöse oder subkutane Anwendungen.

In einer Ausführungsform der Erfindung wird das Arzneimittel in Form einer Tablette, eines Dragées, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt.

Ein weiterer Gegenstand der vorliegenden Erfindung ist die Verwendung des erfindungsgemäßen Verbindung zur Herstellung eines Arzneimittels zur Therapie oder Prophylaxe einer kardiovaskulären Erkrankung oder eines thromboembolischen Ereignisses, insbesondere in oraler, subkutaner, intravenöser oder transdermaler Form verwendet.

Die Erfindung soll nachstehend anhand von mehreren Ausführungsbeispielen näher erläutert werden, ohne sie darauf zu beschränken.

### Methoden

Analytische HPLC: Shimadzu LC-10A System, Säule: Phenomenex-Luna C₁₈, 5 µm (250 x 4 mm) Lösungsmittel A: 0,1 % TFA in Wasser, B: 0,1 % TFA in ACN, Gradient: 10 % B bis 70 % B in 60 min, 1 ml/min Fluß, Detektion bei 220 oder 215 nm.
Präparative HPLC: Shimadzu LC-8A System, Säule: Phenomenex-Luna C18, 5µm (250x 30 mm) Lösungsmittel A: 0,1 % TFA in Wasser, B: 0,1 % TFA in ACN, Gradient: 5 % B bis 50 % B in 120 mm, 10ml/min Fluß, Detektion bei 220 mm.

Massenspektroskopie: Die Massenspektren wurden auf einem ESI-MS LCQ der Firma Finnigan (Bremen, Deutschland), gemessen.

### Verwendete Abkürzungen

- Ac: Acetyl
- AcOxam: N-(Acetyloxy)amidin
- Amb: Amidomethylbenzen
- 4-Amba: 4-Amidinobenzylamid
- Boc: tert.-Butyloxycarbonyl
- Bzl: Benzyl
- Bzls: Benzylsulfonyl
- dCha: d-βCyclohexylalanin
- CKIBE: Chlorkohlensäureisobutylester
- DIEA: Diisopropylethylamin
- DCM: Dichlormethan
- DMF: N,N-Dimethylformamid
- i.V.: im Vakuum
- MS: Massenspektroskopie
- NMM: N-Methylmorpholin
- PyBOP: Benzotriazol-1-yl-N-oxy-tris(pyrrolidino)phosphoniumhexafluorophosphat
- TEA: Triethylamin
- TFA: Trifluoressigsäure
- THF: Tetrahydrofuran
- TMS-Cl: Trimethylsilylchlorid
- tBu: tert.-Butyl

### Beispiel 1

### Bzls-D,L-homoAla(4-Pyr)-Gly-4Amba x 2 TFA

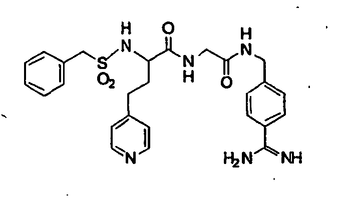

### 1a) H-Gly-4-(Acetyloxamidino)Benzylamid x HCl (H-Gly-Amb(4AcOxam))

2 g (5,49 mmol) Boc-Gly-4-(Acetyloxamidino)Benzylamid (hergestellt wie in WO 01/96286 A2 beschrieben) wurden mit 30 ml 1 N HCl in Eisessig versetzt. Der Ansatz wurde gelegentlich umgeschüttelt. Nach 45 min wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,55 g (5,15 mmol), weißer Feststoff

### 1b) Boc-D,L-homoAla(4-Pyr)-Gly-Amb(4AcOxam)

250 mg (0,89 mmol) Boc-D,L-homoAla(4-Pyr)-OH [RSP Amino Acids DBA, Shirley MA, USA] und 308 mg (1,02 mmol) Produkt 1a wurden in 20 ml DMF gelöst und bei 0 °C mit 531 mg (1,02 mmol) PyBop und 533 µl (3,06 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaClgesättigtem Wasser gewaschen und über Na₂SO₄ getrocknet. Das Lösungsmittel wurde im Vakuum entfernt (gelbliches Öl).
Ausbeute: ca. 600 mg (Rohprodukt), HPLC: 27,89% B

### 1 c) H-D,L-homoAla(4-Pyr)-Gly-Amb(4AcOxam) x HCl

600 mg Rohprodukt 1b wurden mit 10 ml 1N HCl in Eisessig versetzt. Der Ansatz wurde gelegentlich umgeschüttelt. Nach 1 h wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 320 mg (0,69 mmol) hellgelber Feststoff, HPLC: 16,83% B

### 1d) Bzls-D,L-homoAla(4-Pyr)-Gly-Amb(4AcOxam)

75 mg (0,16 mmol) Rohprodukt 1c und 37 mg (0,19 mmol) Phenylmethansulfonylchlorid (Bzls-Cl) [Fluka] wurden in 10 ml DMF gelöst und bei 0 °C mit 68 µl (0,39 mmol) DIEA versetzt. Der Ansatz wurde 20 min bei 0°C und weitere 2 h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaCl- gesättigtem Wasser gewaschen und über Na₂SO₄ getrocknet. Das Lösungsmittel wurde im Vakuum entfernt (helles Öl).
Ausbeute: ca. 280 mg (Rohprodukt), HPLC: 29,27% B

### 1e) Bzls-D,L-homoAla(4-Pyr)-Gly-4Amba

Das Rohprodukt 1d wurde in 50ml 90% Essigsäure gelöst und mit 20 mg Katalysator (10% Pd/C) versetzt. Der Ansatz wurde bei Raumtemperatur und Normaldruck für 5 h mit Wasserstoff hydriert. Anschließend wurde der Katalysator abfiltriert und das Lösungsmittel im Vakuum eingeengt. Der verbleibende Rückstand wurde im Vakuum getrocknet und mit präparativer reversed- phase HPLC gereinigt und lyophilisiert.
Ausbeute: 34,6 mg (0,054 mmol) lyophilisiertes Pulver, HPLC: 22,97% B
MS: berechnet 522,20 (monoisotopic), gefunden 523,4 [M+H]⁺

### Beispiel 2

### 4-PMs-D,L-homoAla(4-Pyr)-Gly-4Amba x 3 Acetat

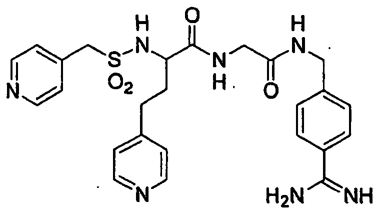

### 2a 4-PMs-D,L-homoAla(4-Pyr)-Gly-Amb(4AcOxam)

50 mg (0,11 mmol) Produkt 1c wurden in 10 ml DCM suspendiert und mit 34 µl (0,28 mmol) Chlortrimethylsilan (= TMS-Cl) [Merck] und 69 µl (0,4 mmol) DIEA versetzt und der Ansatz 15 min bei Raumtemperatur gerührt. Anschließend wurden 41 mg (0,12 mmol) 4-Pyridylmethylsulfonylchlorid x Triflat (= 4-PMs-Cl) [Array Biopharma, Boulder, CO, USA] und weitere 20 µl (0,11 mmol) DIEA zugegeben und weiter über Nacht bei Raumtemperatur gerührt. Dann wurde das Lösungsmittel im Vakuum entfernt. Der Rückstand wurde ohne weitere Reinigung direkt für den nächsten Syntheseschritt eingesetzt.

### 2b)4-PMs-D,L-homoAla(-Pyr)Gly-4Amba

Das Rohprodukt 2a wurde in 50ml 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde in 5 ml Wasser gelöst und auf eine Ionenaustauschsäule (Fractogel- EMD COO- Säule, Dimension 16 x 125 mm, equilibriert mit Wasser) aufgegeben. Die Säule wurde mit 85 ml Wasser gewaschen und anschließend das Produkt mit einem Ammoniumacetat- Gradienten eluiert. Die Produkt-enthaltenden Fraktionen (HPLC-Kontrolle) wurden vereinigt und lyophilisiert.
Ausbeute: 20 mg (0,034 mmol) lyophilisiertes Pulver, HPLC: 13,14% B
MS: berechnet 523,20 (monoisotopic), gefunden 524,3 [M+H]⁺

### Beispiel 3

### Bzls-D,L-homoAla(4-Pyr)-Ser-4Amba x 2 TFA

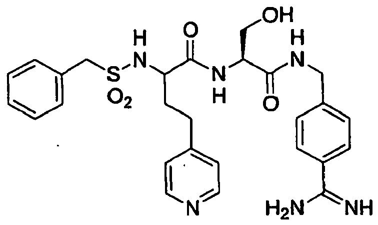

### 3a) Boc-4-Cyanobenzylamid

100 g (0,593 mol) 4-Cyanobenzylamin x HCl wurden in 1,21 Dioxan und 600 ml 2N NaOH gelöst. 142,3 g (0,652 mol) di(tert.-Butyl)Pyrocarbonate wurden bei 0 °C über 10 min in zwei Portionen zugegeben. Der pH-Wert wurde durch Zugabe von 2 N NaOH auf 9-10 eingestellt und der Ansatz für weitere 4 h gerührt. Das Lösungsmittel wurde im Vakuum entfernt, der Rückstand mit Essigester aufgenommen, jeweils 3x mit 5% KHSO₄ und NaCl- gesättigtem Wasser gewaschen und anschließend über Na₂SO₄ getrocknet. Das Lösungsmittel wurde im Vakuum entfernt (weißer Feststoff).
Ausbeute: 132,6 g (0,57 mol) weißer Feststoff, HPLC: 51,6% B

### 3b) Boc-4-Acetyloxamidinobenzylamid

130 g (0,56 mol) Produkt 3a, 58,4 g (0,84 mol) Hydroxylamin x HCl und 146 ml DIEA wurden in 1,51 Methanol gelöst. Der Ansatz wurde 6 h unter Rückfluß gekocht und anschließend über Nacht bei Raumtemperatur gerührt. Das Lösungsmittel wurde im Vakuum entfernt, der ölige Rückstand in 1,51 Essigsäure gelöst, mit 160 ml (1,68 mol) Acetanhydrid versetzt und 30 min gerührt. Das Lösungsmittel wurde im Vakuum entfernt, der Rückstand mit Essigester aufgenommen und 3x mit NaCl- gesättigtem Wasser gewaschen und anschließend über Na₂SO₄ getrocknet. Das Lösungsmittel wurde im Vakuum weitestgehend entfernt und das Produkt aus Essigester kristallisiert.
Ausbeute: 110,6 g (0,36 mol) kristalliner Feststoff, HPLC: 39,76% B

### 3c) H-4-Acetyloxamidinobenzylamin x HCl

50 g (163 mmol) Produkt 3b wurden in 11 Essigsäure gelöst und es wurden 800 ml 1N HCl in Eisessig zugegeben. Der Ansatz wurde geschüttelt und nach einigen Minuten begann das Produkt auszufallen. Nach 75 min wurde das Produkt abgesaugt, mit Diethylether gewaschen und im Vakuum getrocknet.
Ausbeute: 36 g (147,7 mmol) weißer Feststoff, HPLC: 18,97% B

### 3d) Boc-Ser-4-Acetyloxamidinobenzylamid

25 g (122 mmol) Boc-Ser-OH wurden in 750 ml DMF gelöst und auf -15°C gekühlt. Es wurden 13,42 ml (122 mmol) N-Methylmorpholin und 15,86 ml (122 ml) Chlorkohlensäureisobutylester zugegeben und 10 min gerührt. Dann wurden 29,74 g (122 mmol) Produkt 3c und 13,42 ml (122 mmol) N-Methylmorpholin hinzugefügt und der Ansatz für 1 h bei -15 °C und über Nacht bei Raumtemperatur gerührt. Anschließend wurde das DMF im Vakuum entfernt, der Rückstand in 21 Essigester gelöst und jeweils 2x mit 300 ml gesättigter NaHCO₃- Lösung und 300 ml NaCl- gesättigtem Wasser gewaschen, über Na₂SO₄ getrocknet und das Lösungsmittel im Vakuum entfernt (Öl).
Ausbeute: 43 g Rohprukt Öl, HPLC: 29,87% B

### 3e) H-Ser-4-Acetyloxamidinobenzylamid x TFA

40 g des öligen Rohproduktes 3d wurden mit 200 ml Trifluoressigsäure versetzt und 1 h gerührt. Das Produkt wurde durch Zugabe von Diethylether gefällt, abgesaugt, mit Diethylether gewaschen und im Vakuum getrocknet.
Ausbeute: 27 g (66 mmol) weißer Feststoff, HPLC: 20,22% B

### 3f) Boc-D,L-homoAla(4-Pyr)-Ser-Amb(4AcOxam)

100 mg (0,36 mmol) Boc-D,L-homoAla(4-Pyr)-OH [RSP Amino Acids DBA, Shirley MA, USA] und 161 mg (0,4 mmol) Rohprodukt 3e wurden in 15 ml DMF gelöst und bei 0 °C mit 206 mg (0,4 mmol) PyBop und 207 µl (1,2 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaClgesättigtem Wasser gewaschen und über Na₂SO₄ getrocknet. Das Lösungsmittel wurde im Vakuum entfernt (helles Öl).
Ausbeute: ca. 300 mg (Rohprodukt), HPLC: 26,8% B und 27,4% B (Doppelpeak, Racemat)

### 3g) H-D,L-homoAla(4-Pyr)-Ser-Amb(4AcOxam) x TFA

300 mg Rohprodukt aus 3f wurden in 5 ml 50% TFA in Dichlormethan versetzt. Der Ansatz wurde gelegentlich umgeschüttelt. Nach 45 min wurde das Lösungsmittel eingeengt, der Rückstand in Methanol angelöst und das Produkt durch Zugabe von Diethylether gefällt, auf einer Fritte abgesaugt, mit Ether gewaschen und im Vakuum getrocknet.
Ausbeute: 186 mg (0,33 mmol) weißer Feststoff, HPLC: 21,6% B und 22,7% B (Doppelpeak, Racemat)

### 3h) Bzls-D,L-homoAla(4-Pyr)-Ser-Amb(4AcOxam)

75 mg (0,13 mmol) Produkt 3g und 38 mg (0,2 mmol) Phenylmethansulfonylchlorid (= Bzls-Cl) [Fluka] wurden in 10 ml DMF gelöst und bei 0 °C mit 68 µl (0,39 mmol) DIEA versetzt. Der Ansatz wurde 20 min bei 0°C und über Nacht bei Raumtemperatur gerührt. Das Lösungsmittel wurde im Vakuum entfernt (Öl).
Ausbeute: ca. 120 mg (Rohprodukt), HPLC: 28,1% B und 28,6% B (Doppelpeak)

### 3i) Bzls-D,L-homoAla(4-Pyr-Ser-4Amba x 2 TFA

Das Rohprodukt aus 3h wurde in 50ml 90% Essigsäure gelöst und mit 20 mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde im Vakuum getrocknet und mit präparativer reversed- phase HPLC gereinigt und lyophilisiert. Dadurch konnten die Diastereomere getrennt werden.
HPLC: 22,01% B (Verbindung 3a) und 22,6% B (Verbindung 3b).
MS: berechnet 552,22 (monoisotopic), gefunden 553,5 [M+H]⁺

### Beispiel 4

### 4-PMs-D,L-homoAla(4-Pyr)-Ser-4Amba x 3 Acetat

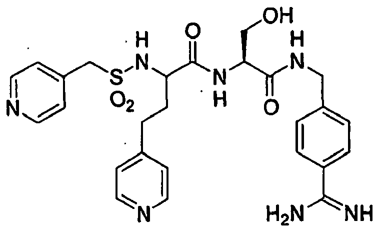

### 4a) 4-PMs-D,L-homoAla(4-Pyr)Ser-Amb(4AcOxam)

55 mg (0,1 mmol) Produkt 3g wurden in 10 ml DCM suspendiert und mit 31 µl (0,25 mmol) Chlortrimethylsilan (= TMS-Cl) [Merck] und 61 µl (0,36 mmol) DIEA versetzt und der Ansatz 15 min bei Raumtemperatur gerührt. Anschließend wurden 36 mg (0,105 mmol) 4-Pyridylmethylsulfonylchlorid x Triflat (= 4-PMs-Cl) [Array Biopharma, Boulder, CO, USA] und weitere 17,5 µl (0,1 mmol) DIEA zugegeben und weiter über Nacht bei Raumtemperatur gerührt. Dann wurde das Lösungsmittel im Vakuum entfernt. Der Rückstand wurde ohne weitere Reinigung direkt für den nächsten Syntheseschritt eingesetzt.

### 4b) 4-PMs-D,L-homoAla(4-Pyr)-Ser-4Amba x 3 Acetat

Das Rohprodukt aus 4a wurde in 50ml 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde in 5 ml Wasser gelöst und auf eine Ionenaustauschsäule (Fractogel- EMD COO- Säule, Dimension 16 x 125 mm, equilibriert mit Wasser) aufgegeben. Die Säule wurde mit 85 ml Wasser gewaschen und anschließend das Produkt mit einem Ammoniumacetat- Gradienten eluiert. Die Produkt-enthaltenden Fraktionen wurden vereinigt und lyophilisiert.
Ausbeute: 17,2 mg (0,028 mmol) lyophilisiertes Pulver, HPLC: 12,1 undd 12,3% B (Doppelpeak, Racemat)
MS: berechnet 553,21 (monoisotopic), gefunden 554,5 [M+H]⁺

### Beispiel 5

### Bzls-d-homoTyr-Gly-4Amba x TFA

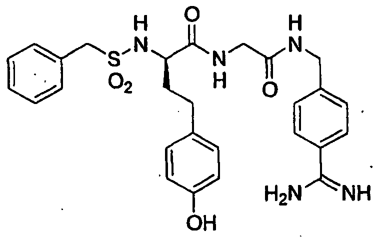

### 5a) Bzls-d-homoTyr-OH

300 mg (1,09 mmol) H-d-homoTyr-OH x HBr [Chem-Impex International, Wood Dale, IL, USA] wurden in 20 ml DCM suspendiert und mit 425 µl (3,37 mmol) Chlortrimethylsilan (= TMS-Cl) [Merck] und 586 µl (3,37 mmol) DIEA versetzt und der Ansatz 1 h bei 60 °C unter Rückfluß gerührt, dann wieder auf Raumtemperatur abgekühlt. Anschließend wurden 229 mg (1,2 mmol) Phenylmethansulfonylchlorid (= Bzls-Cl)
[Fluka] und weitere 190 µl (1,09 mmol) DIEA zugegeben und für 2 h bei Raumtemperatur gerührt. Das Lösungsmittel wurde im Vakuum entfernt, der Rückstand in Essigester aufgenommen, 1x mit 5% KHSO₄- Lösung und 2x mit NaCl- gesättigtem Wasser gewaschen und über Na₂SO₄ getrocknet. Nach Entfernen des Lösungsmittels wurde das Produkt aus Essigester kristallisiert.
Ausbeute: 353 mg (1,01 mmol) hellgelber Feststoff, HPLC: 40,9% B

### 5b) Bzls-d-homoTyr-Gly-Amb(4AcOxam)

50 mg (0,14 mmol) Produkt 5a und 43 mg (0,14 mmol) H-Gly-Amb(4AcOxam) (= Produkt 1a) wurden in 15 ml DMF gelöst und bei 0 °C mit 74,4 mg (0,14 mmol) PyBop und 74,6 µl (0,43 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, l x mit 5% KHSO₄- Lösung und 2x mit NaClgesättigtem Wasser gewaschen und über Na₂SO₄ getrocknet. Als Rückstand blieb ein helles Öl.
Ausbeute: ca. 200 mg (Rohprodukt), HPLC: 39,84% B

### 5c) Bzls-d-homoTyr-Gly-4Amba

Das Rohprodukt aus 5b wurde in 50ml 90% Essigsäure gelöst und mit 20 mg Katalysator (10% Pd/C) versetzt. Der Ansatz wurde bei Raumtemperatur und Normaldruck für 6 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 37,5 mg (0,058 mmol) lyophilisiertes Pulver, HPLC: 32, 37% B
MS: berechnet 537,20 (monoisotopic), gefunden 538,4 [M+H]⁺

### Beispiel 6

### Bzls-d-homoTyr-Ser-4Amba x TFA

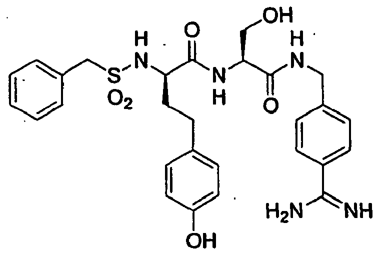

### 6a) Bzls-d-homoTyr-Ser-Amb(4AcOxam)

50 mg (0,14 mmol) Produkt 5a und 58,4 mg (0,14 mmol) H-Ser-Amb(4AcOxam) (= Produkt 3e) wurden in 15 ml DMF gelöst und bei 0 °C mit 74,4 mg (0,14 mmol) PyBop und 74,6 µl (0,43 mmol) DIEA versetzt. Der Ansatz wurde 20 min bei 0°C und über Nacht bei Raumtemperatur gerührt. Anschließend wurde das Lösungsmittel im Vakuum entfernt, der Rückstand in Essigester aufgenommen, 1x mit 5% KHSO₄- Lösung und 2x mit NaClgesättigtem Wasser gewaschen und über Na₂SO₄ getrocknet (helles Öl).
Ausbeute: ca. 165 mg (Rohprodukt), HPLC: 38,49% B

### 6b) Bzls-d-homoTyr-Ser-4Amba x TFA

Das Rohprodukt aus 6a wurde in 50ml 90% Essigsäure gelöst und mit 20 mg Katalysator (10% Pd/C) versetzt. Der Ansatz wurde bei Raumtemperatur und Normaldruck für 6 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 38 mg (0,056 mmol) lyophilisiertes Pulver, HPLC: 31,74% B
MS: berechnet 567,22 (monoisotopic), gefunden 568,5 [M+H]⁺

### Beispiel 7

### 4-PMs-dhomoPhe-Gly-4Amba x 2 TFA

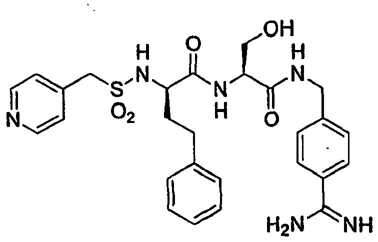

### 7a) Boc-d-homoPhe-Gly-Amb(4AcOxam)

732 mg (2,62 mmol) Boc-d-homoPhe-OH [Bachem] und 788 mg (2,62 mmol) H-Gly-Amb(4AcOxam) (=Produkt 1a) wurden in 50 ml DMF gelöst und bei 0 °C mit 1,36 g (2,62 mmol) PyBop und 1,37 ml (7,86 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, jeweils 2x mit 5% KHSO₄, gesättigter NaHCO₃-Lösung und NaCl- gesättigtem Wasser gewaschen und anschließend über Na₂SO₄ getrocknet. Das Lösungsmittel wurde im Vakuum entfernt (hellbraunes Öl).
Ausbeute: ca. 1,8 g (Rohprodukt), HPLC: 47,87% B

### 7b) H-d-homoPhe-Gly-Amb(4AcOxam) x HCl

600 mg Rohprodukt aus 7a wurden mit 15 ml 1N HCl in Eisessig versetzt. Der Ansatz wurde gelegentlich umgeschüttelt. Nach 1 h wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,02g (2,2 mmol) hellgelber Feststoff, HPLC: 28,11% B

### 7c)4-PMs-dhomoPhe-Gly-Amb(4AcOxam)

50 mg (0,11 mmol) Produkt 7b wurden in 10 ml DCM suspendiert und mit 20,5 µl (0,16 mmol) Chlortrimethylsilan (= TMS-Cl) [Merck] und 49 µl (0,28 mmol) DIEA versetzt und der Ansatz 15 min bei Raumtemperatur gerührt. Anschließend wurden 41 mg (0,12 mmol) 4-Pyridylmethylsulfonylchlorid x Triflat (= 4-PMs-Cl) [Array Biopharma, Boulder, CO, USA] und weitere 20 µl (0,11 mmol) DIEA zugegeben und weiter für 2 h bei Raumtemperatur gerührt. Dann wurde das Lösungsmittel im Vakuum entfernt. Der Rückstand wurde ohne weitere Reinigung direkt für den nächsten Syntheseschritt eingesetzt

### 7d) 4-PMs-dhomoPhe-Gly-4Amba x 2 TFA

Das Rohprodukt aus 7c wurde in 50ml 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 30 mg (0,047 mmol) lyophilisiertes Pulver, HPLC: 26,01% B
MS: berechnet 522,20 (monoisotopic), gefunden 523,3 [M+H]⁺

### Beispiel 8

### 2-PMs-d-homoPhe-Gly-4Amba x 2 TFA

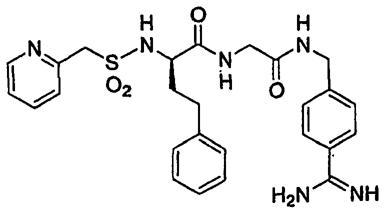

### 8a) 2-PMs-dhomoPhe-OH

75 mg (0,42 mmol) H-d-homoPhe-OH [Bachem] wurden in 10 ml DCM suspendiert und mit 116 µl (0,92 mmol) Chlortrimethylsilan (= TMS-Cl) [Merck] und 160 µl (0,92 mmol) DIEA versetzt und der Ansatz 1h bei 60 °C unter Rückfluß gerührt, dann wieder auf Raumtemperatur abgekühlt. Anschließend wurden 150 mg (0,44 mmol) 2-Pyridylmethylsulfonylchlorid x Triflat (= 2-PMs-Cl) [Array Biopharma, Boulder, CO, USA] und weitere 77 µl (0,44 mmol) DIEA zugegeben und weiter über Nacht bei Raumtemperatur gerührt. Dann wurde das Lösungsmittel entfernt. Der Rückstand wurde ohne weitere Reinigung direkt für den nächsten Syntheseschritt eingesetzt.
Ausbeute: ca. 300 mg Rohprodukt, HPLC: 32,86% B

### 8b) 2-PMs-dhomoPhe-Gly-Amb(4AcOxam)

150 mg (ca. 0,2 mmol) Rohprodukt 8a und 60,2 mg (0,2mmol) H-Gly-Amb(4AcOxam (=Produkt 1a) wurden in 10 ml DMF gelöst und bei 0 °C mit 104 mg (0,2 mmol) PyBop und 104,5 µl (0,6 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 2x mit gesättigter NaHCO₃-Lösung und 2x mit NaCl- gesättigtem Wasser gewaschen und über Na₂SO₄ getrocknet. Das Lösungsmittel wurde im Vakuum entfernt (hellbraunes Öl).
HPLC: 35,28% B

### 8c) 2-PMs-dhomoPhe-Gly-4Amba

Das Rohprodukt aus 8b wurde in 50ml 90% Essigsäure gelöst und mit 20 mg Katalysator (10% Pd/C) versetzt. Der Ansatz wurde bei Raumtemperatur und Normaldruck für 5 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und das Produkt lyophilisiert.
Ausbeute: 69 mg (0,11 mmol) lyophilisiertes Pulver, HPLC: 31,18% B
MS: berechnet 522,20 (monoisotopic), gefunden 523,4 [M+H]⁺

### Beispiel 9

### 3-PMs-dhomoPhe-Gly-4Amba x 2 TFA

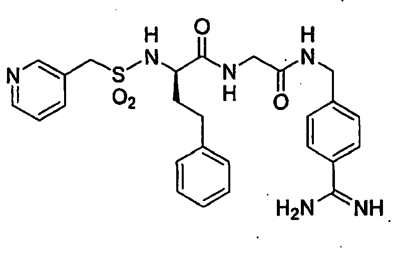

### 9) 3-PMs-dhomoPhe-Gly-4Amba

Beispiel 9 wurde in Analogie zu Beispiel 8 synthetisiert, nur dass 3-Pyridylmethylsulfonylchlorid x Triflat (= 3-PMs-Cl) [Array Biopharma, Boulder, CO, USA] verwendet wurde. Das Endprodukt wurde mit präparativer reversed- phase HPLC gereinigt und lyophilisiert.
Ausbeute: 62 mg (0,097 mmol) lyophilisiertes Pulver, HPLC: 29,08% B
MS: berechnet 522,20 (monoisotopic), gefunden 523,4 [M+H]⁺

**Tabelle 1: Bestimmung der Hemmkonstanten für Faktor Xa und Thrombin. Zusätzlich ist das Selektivitätsverhältnis SV angegeben (SV = Kᵢ Thrombin/Ki Faktor Xa).**

| Nr. | Struktur | Kᵢ (µM) | | SV |
|---|---|---|---|---|
| | | Faktor Xa | Thrombin | |
| 10 | 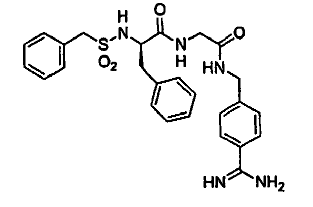 | 0,026 | 0,068 | 2,6 |
| 11 | 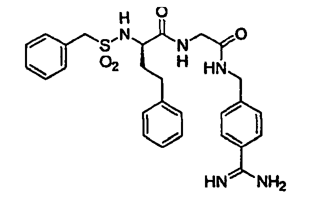 | 0,0065 | 0,047 | 7,2 |
| 12 | 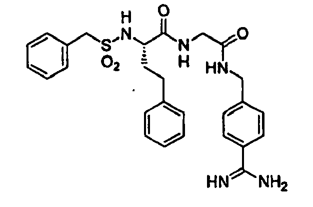 | 0,36 | 11 | 31 |
| 13 | 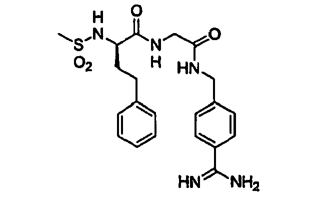 | 1,1 | 1,3 | 1,2 |
| 8 | 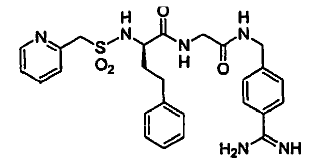 | 0, 051 | 4,9 | 96 |
| 9 | 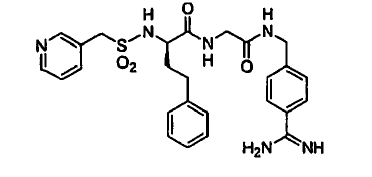 | 0,062 | 5,9 | 95 |
| 14 | 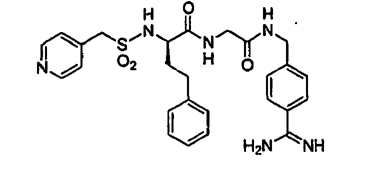 | 0,08 | 3,6 | 45 |
| 15 | 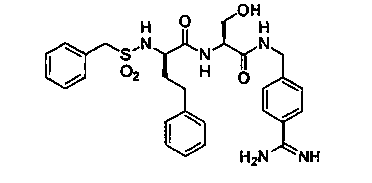 | 0,04 | 0,6 | 15 |
| 7 | 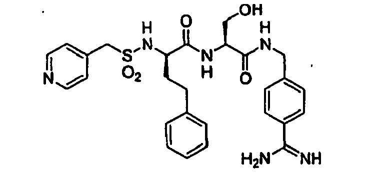 | 0,46 | 3,3 | 7,2 |
| 16 | 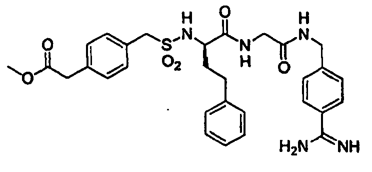 | 0,038 | 1,8 | 47 |
| 17 | 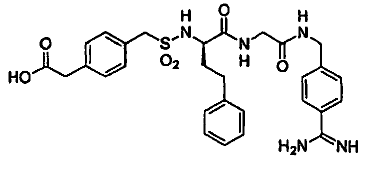 | 0,054 | 14 | 259 |
| 18 | 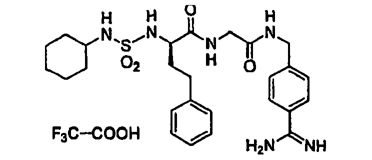 | 0,11 | 5,4 | 49 |
| 19 | 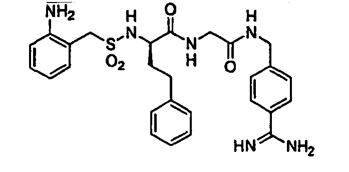 | 0,0067 | 0,92 | 137 |
| 20 | 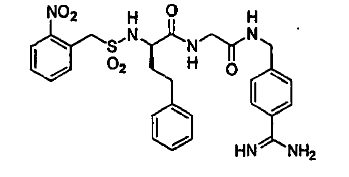 | 0,026 | 1,2 | 46 |
| 5 | 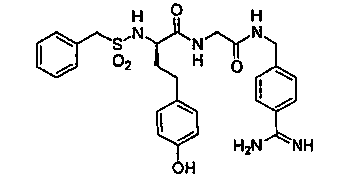 | 0,0027 | 1,5 | 556 |
| 6 | 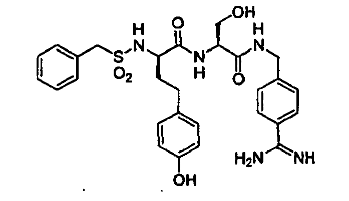 | 0,019 | 1, 4 | 74 |
| 1 | 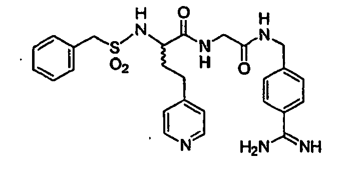 | 0,0029 | 2 | 690 |
| 2 | 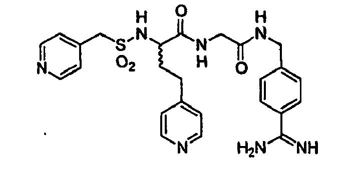 | 0,013 | 3,2 | 246 |
| 3a | 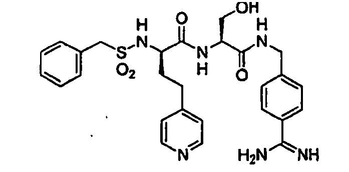 | 0,0094 | 0,91 | 97 |
| 3b | 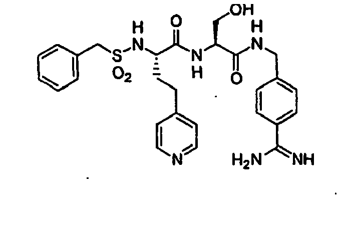 | 0,095 | 4, 6 | 48,4 |
| 4 | 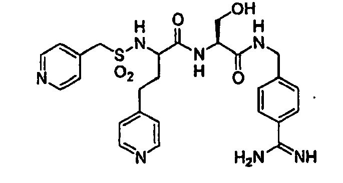 | 0,097 | 6,3 | 65 |
| 21 | 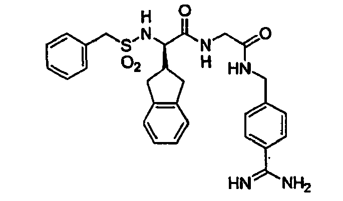 | 0,029 | 0,15 | 5,2 |
| | | | | |
| 22 | 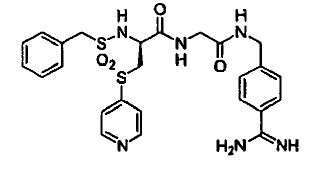 | 0,0027 | 0,7 | 259 |
| 23 | 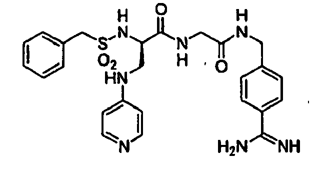 | 0,022 | 2,8 | 127 |
| 24 | 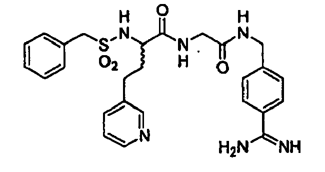 | 0,005 | 2,0 | 400 |
| 25 | 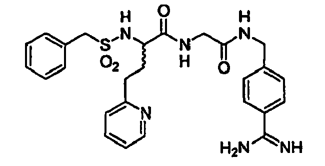 | 0,0021 | 2,0 | 952 |
| 26 | 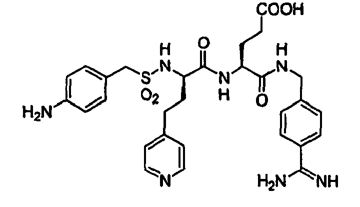 | 0,0017 | 25 | 14705 |
| 27 | 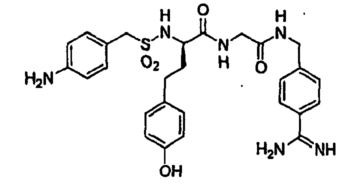 | 0,0019 | 0,56 | 295 |
| 28 | 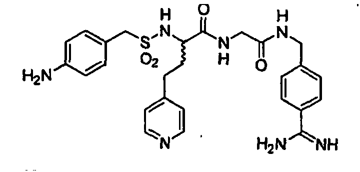 | 0,0022 | 1 | 454 |
| 29 | 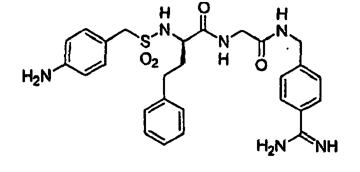 | 0,0026 | 0,26 | 100 |
| 30 | 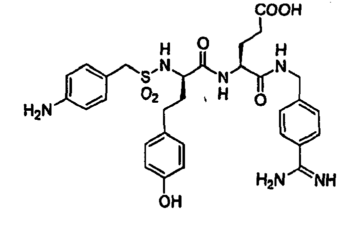 | 0,0034 | 78 | 22940 |
| 31 | 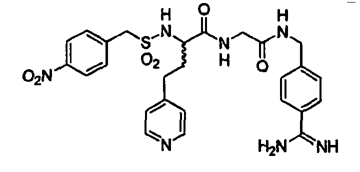 | 0,0035 | 1,9 | 543 |
| 32 | 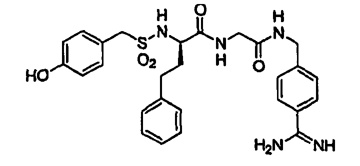 | 0,0036 | 0,38 | 105 |
| 33 | 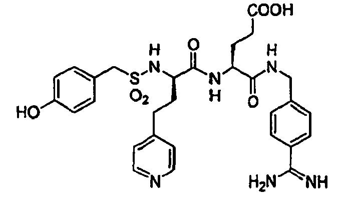 | 0,0036 | 100 | 27778 |
| 34 | 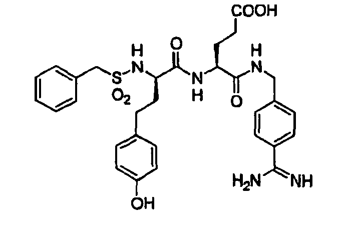 | 0,0037 | 19 | 5135 |
| 35 | 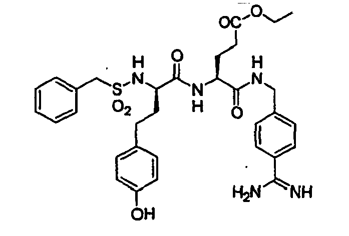 | 0, 005 | 1 | 200 |
| 36 | 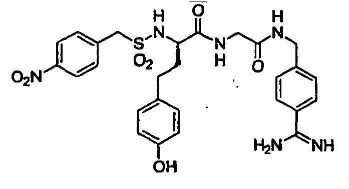 | 0,0052 | 0,86 | 165 |
| 37 | 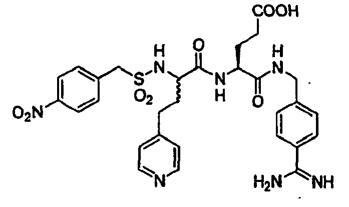 | 0,0056 | 35 | 6250 |
| 38 | 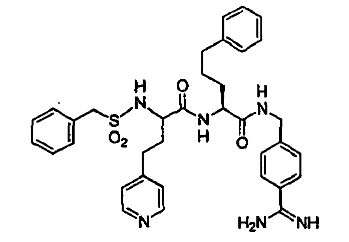 | 0,006 | 0,18 | 30 |
| 39 | 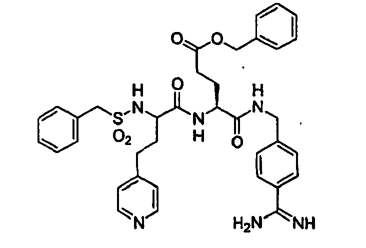 | 0,0064 | 0,17 | 26 |
| 40 | 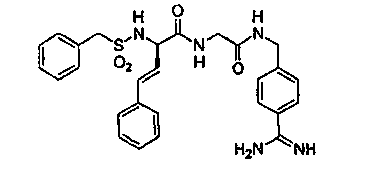 | 0,0065 | 1,1 | 170 |
| 41 | 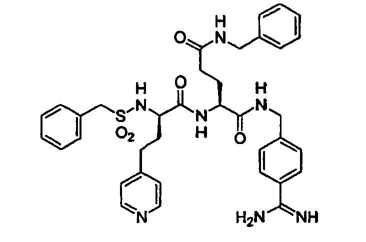 | 0,0068 | 1,7 | 250 |
| 42 | 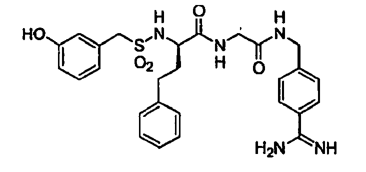 | 0,0072 | 1,5 | 288 |
| 43 | 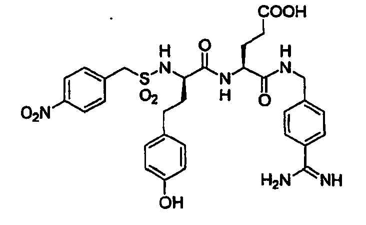 | 0,0075 | 15 | 2000 |
| 44 | 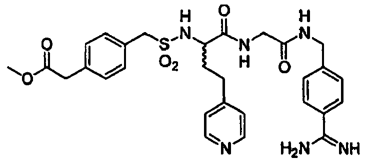 | 0,0082 | 3,8 | 463 |
| 45 | 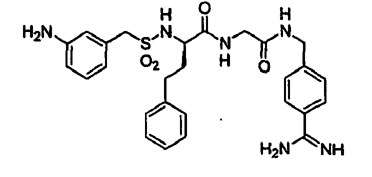 | 0,0093 | 1,4 | 150 |
| 46 | 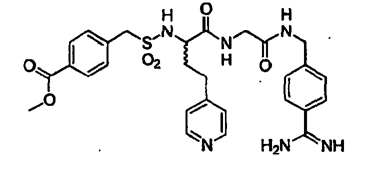 | 0,0098 | 7,6 | 775 |
| 47 | 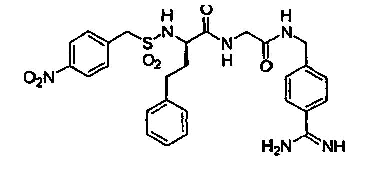 | 0,01 | 0,71 | 71 |
| 48 | 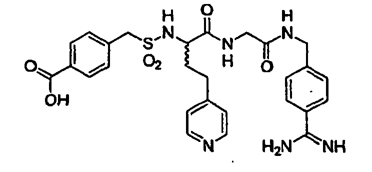 | 0,01 | n.b.* | - |
| 49 | 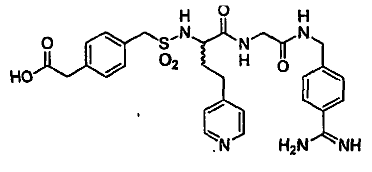 | 0,013 | 38 | 2923 |
| 50 | 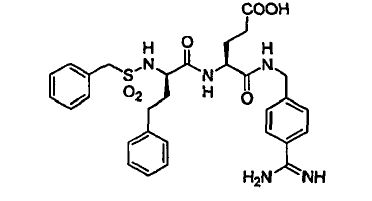 | 0,013 | 15 | 1153 |
| 51 | 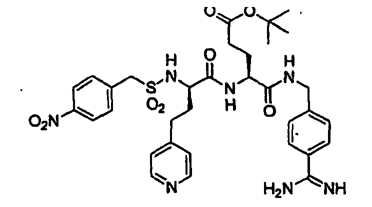 | 0,016 | 1,4 | 87 |
| 52 | 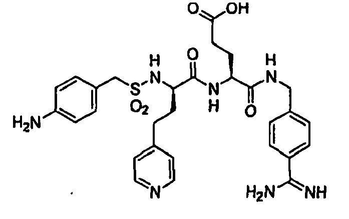 | 0,016 | 84 | 5250 |
| 53 | 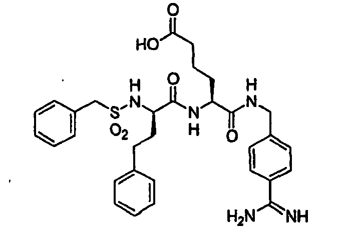 | 0,03 | 0,8 | 27 |
| 54 | 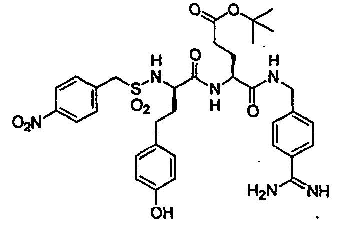 | 0,039 | 0,69 | 18 |
| 55 | 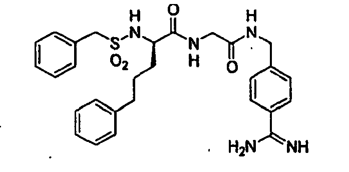 | 0,067 | 0,21 | 3 |
| 56 | 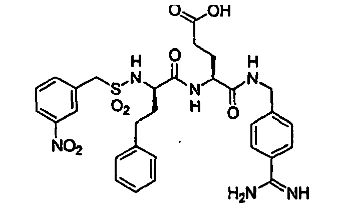 | 0,083 | 13 | 156 |
| 57 | 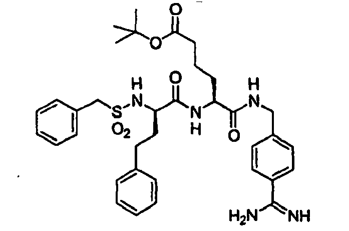 | 0,13 | 0,46 | 3,5 |
| 58 | 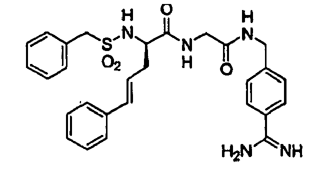 | 0,58 | 1,8 | 3,1 |
| 59 | 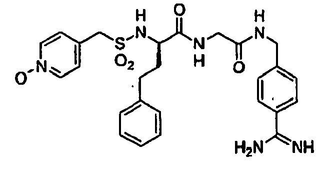 | 0,97 | 16 | 16 |
| 60 | 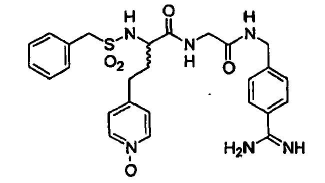 | 0,0048 | 3,5 | 730 |
| | | | | |
| 61 | 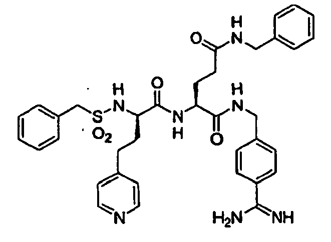 | 0,0068 | 1,7 | 250 |
| 62 | 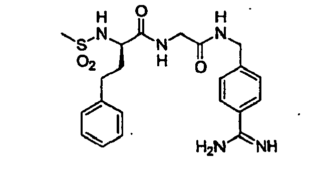 | 1,06 | 1,3 | 1,2 |
| 63 | 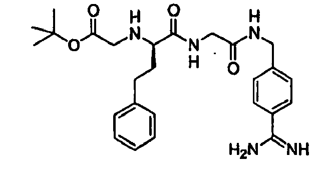 | 0,62 | 1,5 | 2,4 |
| 64 | 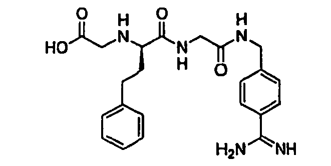 | 0,87 | 28 | 32 |
| 65 | 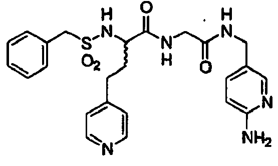 | 0,12 | 100 | 833 |

| | | | | |
|---|---|---|---|---|
| n.b. = nicht bestimmt | | | | |

### Bestimmung der Hemmwirkung

Zur Bestimmung der Hemmwirkung wurden 200 µl Tris-Puffer (0,05 M, 0,154 M NaCl, 5% Ethanol, pH 8,0; enthält den Inhibitor), 25 µl Substrat (Moc-D-Nle-Gly-Arg-pNA in H₂O; Pentapharm Ltd., Basel, Schweiz) und 50 µl Faktor Xa (vom Rind, Diagnostic Reagents Ltd, Thame, GB) bei 25 °C inkubiert. Nach 3 min wurde die Reaktion durch Zugabe von 25 µl Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen. Die Bestimmung der Thrombinhemmung erfolgte analog einer früher beschriebenen Methode (Stürzebecher et al., J. Med. Chem. 40,3091-3099,1997).

## Patentansprüche

1. Verbindung der allgemeinen Formel I 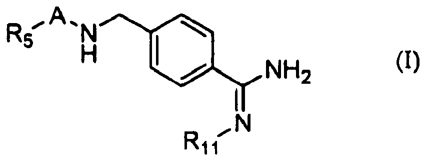 wobei
A P₂—P₁ ist, mit
P₁= 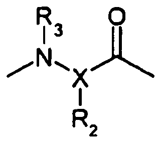 und
P2 = 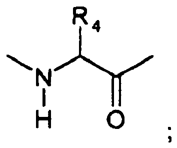
X ein N oder CH, vorzugsweise ein CH;
R₂ ein H, -CH₂-OR₇ oder -CH₂-OCOOR₇ ist, wobei R₇ ein H oder ein verzweigter oder unverzweigter Alkylrest mit 1-5, insbesondere 1-3 C-Atomen, ist, oder R₂ ist ein -CH₂-CH₂-COOR₇*, wobei R₇* ein H oder ein verzweigter oder unverzweigter Alkylrest mit 1-5 C-Atomen, vorzugsweise Ethyl, ist;
R₃ ein H ist;
R₄ -(CH_{2)f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Aryl- oder Heteroarylrest ist, wobei der Aryl- oder Heteroarylrest vorzugsweise 5 bis 14, insbesondere 5 bis 6 C-Atome im Ring und im Falle des Heteroarylrestes vorzugsweise 1 bis 3 N als Heteroatome besitzt, und wobei P₂ in der Struktur A der allgemeinen Formel I in der D- oder L-Konfiguration, vorzugsweise in der D-Konfiguration vorliegt;
R₅ -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R₉*, -SO₂-NH-R₉*, wobei R₉* ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₁, mit R₇ wie oben definiert, -NH₂, -NO₂, -COOR₁₀, -CH₂-COOR₁₀-Gruppe oder ein Cl-, F- oder Br-Atom ist und wobei R₁₀ ein H oder ein Alkylrest mit 1 bis 6, insbesondere mit 1 bis 4 C-Atomen, vor allem Ethyl, ist;
R₁₁ H, OH, oder -COOR₁₂ ist, wobei R₁₂ ein verzweigter oder unverzweigter Alkylrest mit 1 bis 8, vorzugsweise 1 bis 6 C-Atomen ist;
oder eine Verbindung der allgemeinen Formel I in Form ihres Salzes;
mit Ausnahme folgender Verbindungen:
Benzylsulfonyl-D-hPhe-Ser-4-Amidinobenzylamid,
Benzylsulfonyl-D-hAla(4-Pyridyl)-Ser-4-Amidinobenzylamid,
Benzylsulfonyl-D-hTyr-Ser-4-Amidinobenzylamid,
4-NHᵢ-Benzylsulfonyl-D-hPhe-Gly-4-Amidinobenzylamid,
Benzylsulfonyl-D-Indanylglycyl=Gly-4-Amidinobenzylamid,
Benzylsulfonyl-D/L-hAla(4-Pyridyl)-Gly-4-Amidinobenzylamid und
4-Pyridylmethylsulfonyl-D-hPhe-Gly-4-Amidinobenzylamid,
wobei der C-terminale aromatische Benzamidinrest an 1, 2 oder 3 Positionen zusätzlich mit einem Halogen, insbesondere Fluor oder Chlor, oder einem Methyl-, Ethyl-, Propyl-, Methoxy-, Ethoxy-, oder Propoxyrest subsituiert sein kann.

2. Verbindung nach Anspruch 1, wobei mindestens eine Carboxylgruppe als Ester, bevorzugt als Ethylester, geschützt vorliegt.

3. Verbindung nach Anspruch 1 oder 2 in Form eines Prodrugs, bei dem ein oder mehrere Carboxylgruppen in Form ihrer Alkylester mit einem verzweigten oder unverzweigten Alkyl mit 1-5 C-Atomen, vorzugsweise Ethyl, und/oder bei dem ein oder mehrere Hydroxylgruppen als Carbonate, bei denen der endständige Rest gleich R₇, wie oben definiert vorliegen, und/oder bei dem der Amidino- bzw. Guanidinobenzylaminrest als Hydroxyamidin bzw. Hydroxyguanidin oder als Alkyloxycarbonylderivat vorliegt.

4. Verbindung der allgemeinen Formel I 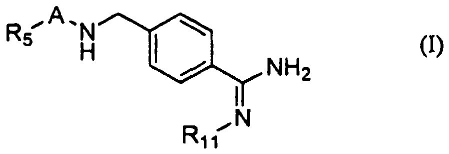 wobei
A P₂—P₁ ist, mit
P₁= 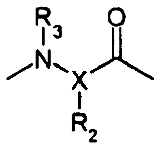 und
P₂ = 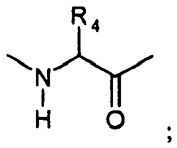
R₂ ein H, -CH₂-CH₂-COOH, -CH₂-CH₂-COOCH₂CH₃ oder -CH₂OH ist;
R₃ ein H ist;
R₄ ein -(CH₂)₂-R₈, -CH₂NHR₈, -(CH₂)₂NHR₈ oder ein -CH₂-4-Hydroxycyclohexylrest ist, wobei R₈ ein einfach oder mehrfach substituierter oder unsubstituierter Aryl- oder Heteroarylrest ist, wobei der Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
R₅ ein Methylsulfonyl, Ethylsulfonyl, n-Propylsulfonyl, iso-Propylsulfonyl, Butylsulfonyl, n-Butylsulfonyl, Benzylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylensulfonyl, N-(oxid)-pyridylmethylensulfonyl, -CH₂-COOH oder ein -CH₂COOCH₂CH₃ Rest ist;
X CH ist;
R₁₁ H, OH oder -COOR₁₂ mit R₁₂ ein verzweigter oder unverzweigter Alkylrest mit 2, 4 oder 6 C-Atomen ist,
oder eine Verbindung der allgemeinen Formel I in Form ihres Salzes;
mit Ausnahme folgender Verbindungen:
Benzylsulfonyl-D-hPhe-Ser-4-Amidinobenzylamid,
Benzylsulfonyl-D-hAla(4-Pyridyl)-Ser-4-Amidinobenzylamid, Benzylsulfonyl-D-hTyr-Ser-4-Amidinobenzylamid,
4-NH₂-Benzylsulfonyl-D-hPhe-Gly-4-Amidinobenzylamid,
Benzylsulfonyl-D-Indanylglycyl-Gly-4-Amidinobenzylamid,
Benzylsulfonyl-D/L-hAla(4-Pyridyl)-Gly-4-Amidinobenzylamid und
4-Pyridylmethylsulfonyl-D-hPhe-Gly-4-Amidinobenzylamid.

5. Verbindung nach mindestens einem der Ansprüche 1 bis 4, dadurch gekenn-zeichnet, dass R₄ ein -CH₂-CH₂-R₈ Rest ist, wobei R₈ ein Aryl- oder Heteroarylrest mit 4-6 Ringatomen ist, der 1 oder 2 Heteroatome, vorzugsweise N, besitzt und der mit einer oder mehreren -NH₂ und/oder -OH Gruppen substituiert ist, vorzugsweise ist P₂ in der Struktur A der allgemeinen Formel I von einem Homophenylalanin, Homotyrosin, Indanylglycin oder 4-Pyridylhomoalanin abgeleitet, und die P₂-Aminosäure liegt insbesondere in der D-Konfiguration vor.

6. Verbindung nach mindestens einem der Ansprüche 1 bis 5, **dadurch gekennzeichnet, dass** der Substituent -OH, -NH₂, -NO₂, -COOH, -COOCH₂CH₃, Fluor, Chlor oder Brom, insbesondere Fluor oder Chlor, ist.

7. Verbindung nach mindestens einem der Ansprüche 1 bis 6, **dadurch gekennzeichnet, dass** die Verbindungen als Salze vorliegen, vorzugsweise mit Mineralsäuren oder mit geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.

8. Verfahren zur Herstellung einer Verbindung gemäß mindestens einem der Ansprüche 1 bis 7, **dadurch gekennzeichnet, dass** sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird.

9. Arzneimittel enthaltend eine Verbindung gemäß mindestens einem der Ansprüche 1 bis 7 sowie pharmazeutisch geeignete Hilfs- und/oder Zusatzstoffe.

10. Arzneimittel nach Anspruch 9, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Emulsion oder Suspension, eines Globuli, Styli, Aerosols, Puders, einer Paste, Creme oder Salbe eingesetzt wird.

11. Verwendung einer Verbindung gemäß mindestens einem der Ansprüche 1 bis 7 zur Herstellung eines Arzneimittels als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.

12. Verbindung der allgemeinen Formel I 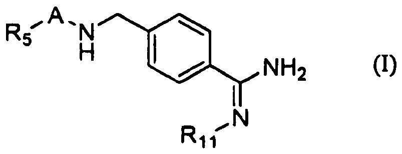 wobei
A P₂—P₁ ist, mit
P₁= 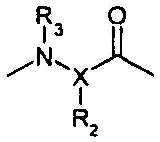 und
P2 = 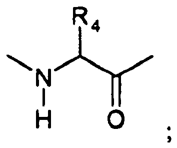
X = N oder CH, vorzugsweise CH;
R₂ eine -(CH₂)ₐCONHR₇* oder -(CH₂)ₐCONHR₇** Gruppe ist, wobei a = 1, 2 oder 3 und
R₇* ein H oder ein verzweigter oder unverzweigter Alkylrest mit 1-5 C-Atomen, vorzugsweise Ethyl;
R₇** ein Arylrest oder ein Heteroarylrest mit 1 -2 Heteroatomen ausgewählt aus N, S oder 0 ist;
R₃ ein H ist;
R₄ -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Aryl- oder Heteroarylrest ist, wobei der Aryl- oder Heteroarylrest 5 bis 14, insbesondere 5 bis 6 C-Atome im Ring und im Falle des Heteroarylrestes vorzugsweise 1 bis 3 N als Heteroatome besitzt, und wobei P₂ in der Struktur A der allgemeinen Formel I in der D-oder L-Konfiguration, vorzugsweise in der D-Konfiguration vorliegt;
R₅ -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R₉*, -SO₂-NH-R₉*, wobei R₉* ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₇, mit R₇ wie oben definiert, -NH₂, -NO_{2,} -COOR₁₀, -CH₂-COOR₁₀-Gruppe oder ein Cl-, F- oder Br-Atom ist und wobei R₁₀ ein H oder ein Alkylrest mit 1 bis 6, insbesondere mit 1 bis 4 C-Atomen, vor allem Ethyl, ist;
R₁₁ H, OH, oder -COOR₁₂ ist, wobei R₁₂ ein verzweigter oder unverzweigter Alkylrest mit 1 bis 8, vorzugsweise 1 bis 6 C-Atomen ist;
oder eine Verbindung der allgemeinen Formel I in Form ihres Salzes.

13. Verbindung nach Anspruch 12, wobei in R₅ R₉ = H ist.

14. Verbindung nach Anspruch 12 oder 13, wobei R₄ eine -CH₂-SR₈ oder -CH₂CH₂-SR₈ Gruppe ist.

15. Verbindung ausgewählt aus folgenden Strukturen:

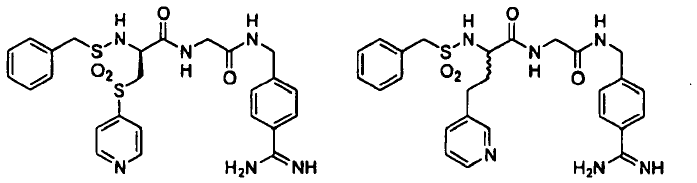

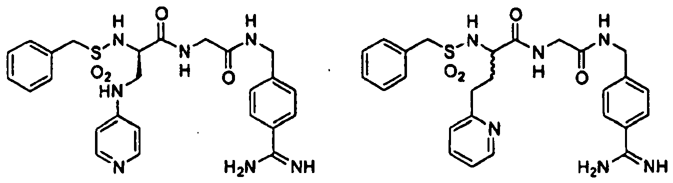

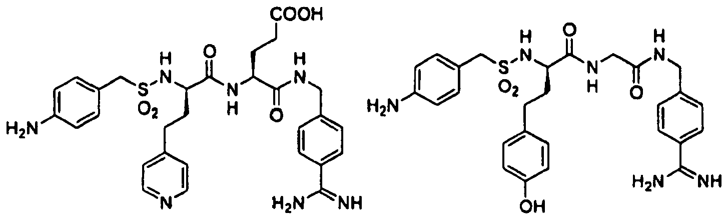

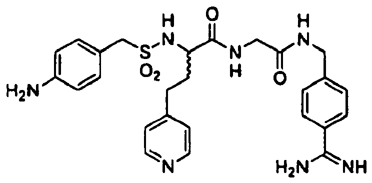

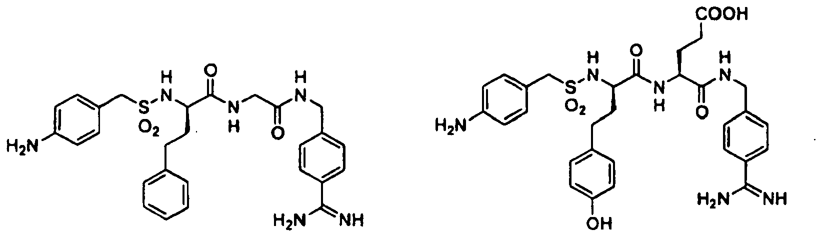

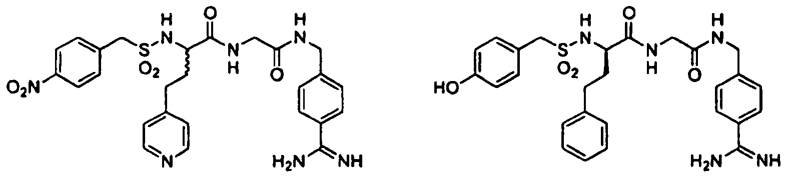

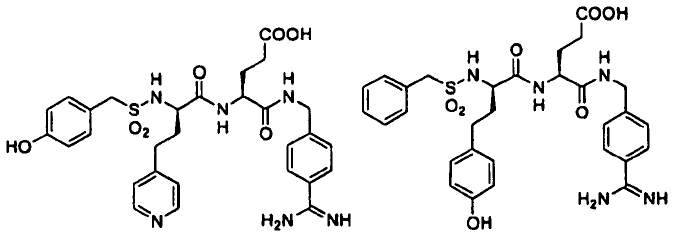

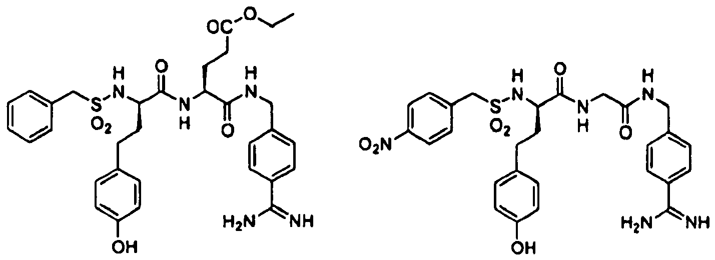

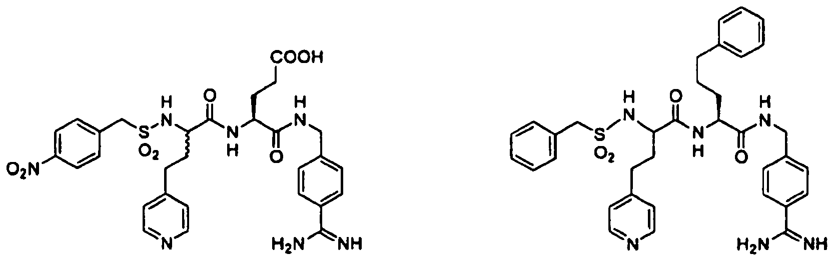

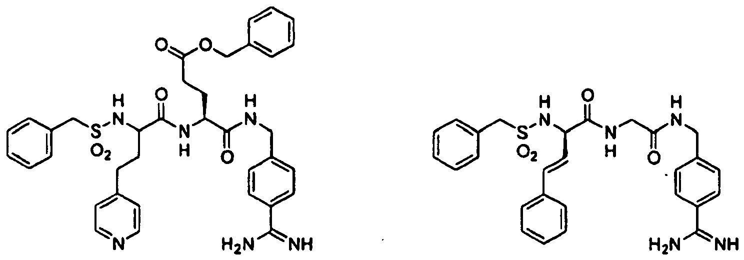

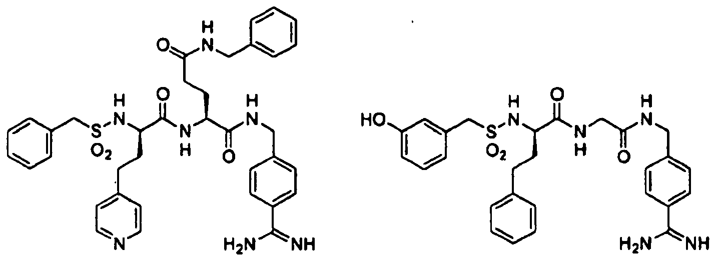

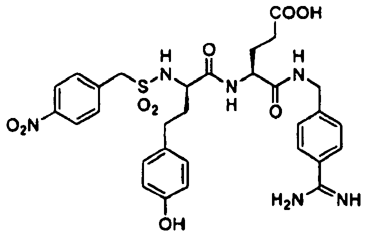

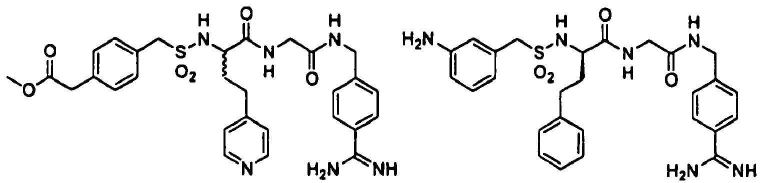

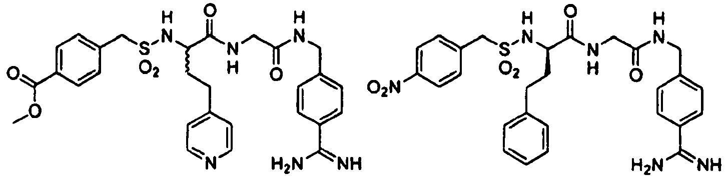

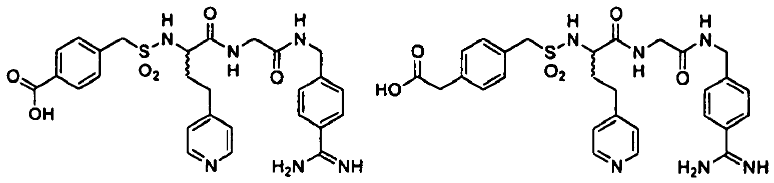

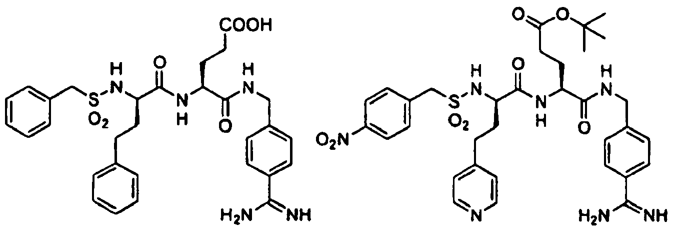

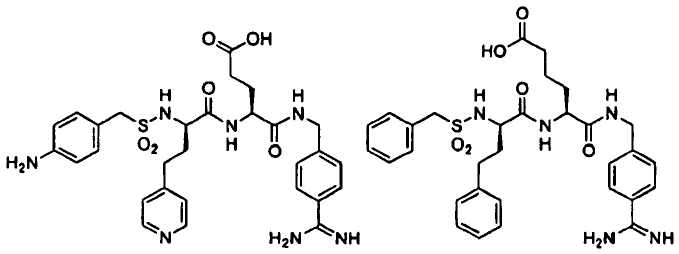

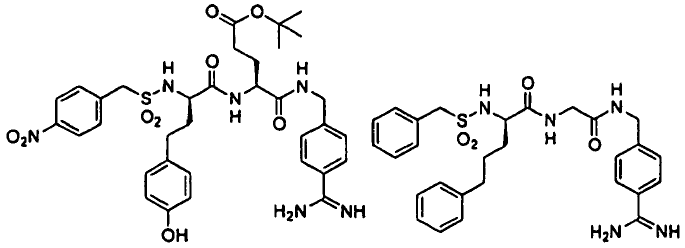

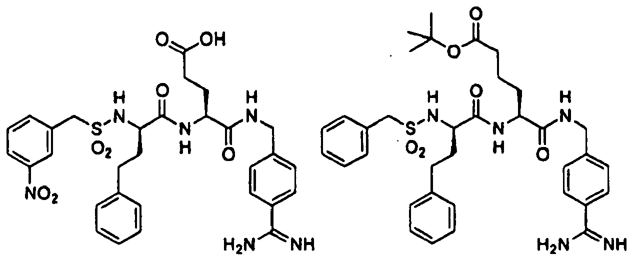

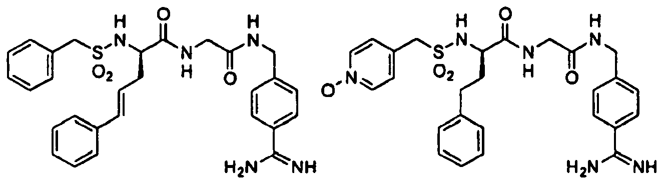

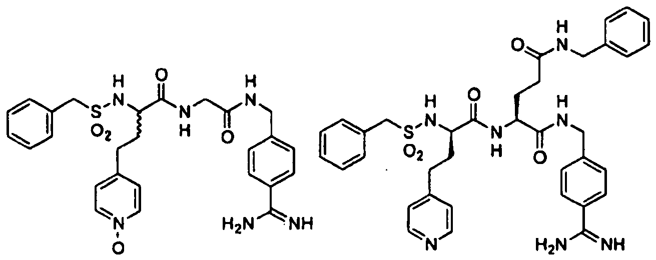

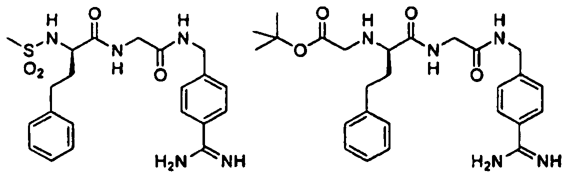

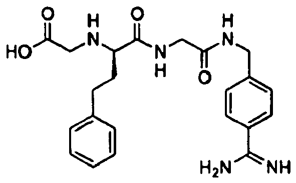

16. Verbindung nach mindestens einem der Ansprüche 12 bis 15, **dadurch gekennzeichnet, dass** die Verbindungen als Salze vorliegen, vorzugsweise mit Mineralsäuren oder mit geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.

17. Verfahren zur Herstellung einer Verbindung gemäß mindestens einem der Ansprüche 12 bis 16, **dadurch gekennzeichnet, dass** sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird.

18. Arzneimittel enthaltend eine Verbindung gemäß mindestens einem der Ansprüche 12 bis 16 sowie pharmazeutisch geeignete Hilfs- und/oder Zusatzstoffe.

19. Arzneimittel nach Anspruch 18, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Emulsion oder Suspension, eines Globuli, Styli, Aerosols, Puders, einer Paste, Creme oder Salbe eingesetzt wird.

20. Verwendung einer Verbindung gemäß mindestens einem der Ansprüche 12 bis 16 zur Herstellung eines Arzneimittels als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.

## Claims

1. A compound of the general formula I 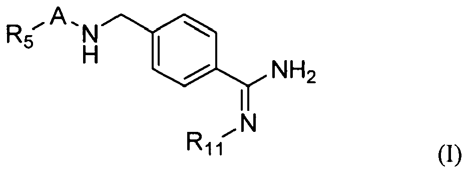 wherein
A is P₂—P₁ with
P₁ = 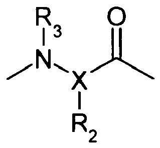 and
P₂ = 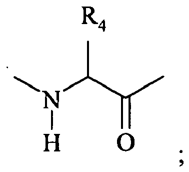
X is an N or a CH, preferably a CH;
R₂ is an H, -CH₂-OR₇ or -CH₂-OCOOR₇, wherein R₇ is an H or a branched or unbranched alkyl radical having 1-5, in particular 1-3 C atoms, or R₂ is a -CH₂-CH₂-COOR₇* wherein R₇* is an H or a branched or unbranched alkyl radical having 1-5 C atoms, preferably ethyl;
R₃ is an H;
R₄ is -(CH₂)_{f}-R₈ with f = 0 or 2, preferably with f = 2, -CH₂NHR₈, -(CH₂)₂NHR₈ or -CH=CH-R₈, wherein R₈ is a mono- or polysubstituted or unsubstituted aryl or heteroaryl radical, wherein the aryl or heteroaryl radical preferably has 5 to 14, in particular 5 to 6 C atoms in the ring and, in the case of the heteroaryl radical, preferably possesses 1 to 3 N as heteroatoms, and wherein P₂ in the structure A of the general formula I is in the D or L configuration, preferably in the D configuration;
R₅ is -(CH₂)i-COOR₉ with i = 1, 2 or 3, preferably with i = 1, and R₉ is equal to a branched or unbranched alkyl radical having 1-5 C atoms, preferably ethyl, or R₅ is -SO₂R₉*, -SO₂-NH-R₉*, wherein R₉* is an H, a branched or unbranched alkyl having 1-10, preferably 1 to 6, in particular 1 to 4, especially 1 to 2 C atoms, a mono- or polysubstituted or unsubstituted aryl, heteroaryl, aralkyl, preferably benzyl, heteroaralkyl radical or a cyclohexylalkyl radical, preferably a cyclohexylmethyl radical, wherein the substituent may be an -OH, -O-COOR₇, -CH₂-OCOOR₇, with R₇ as defined above, -NH₂, -NO₂, -COOR₁₀, -CH₂-COOR₁₀ group or a Cl, F or Br atom, and wherein R₁₀ is an H or an alkyl radical having 1 to 6, in particular having 1 to 4 C atoms, especially ethyl;
R₁₁ is H, OH, or -COOR₁₂, wherein R₁₂ is a branched or unbranched alkyl radical having 1 to 8, preferably 1 to 6 C atoms;
or a compound of the general formula I in the form of its salt;
with the exception of the following compounds:
benzylsulfonyl-D-hPhe-Ser-4-amidino benzylamide,
benzylsulfonyl-D-hAla(4-pyridyl)-Ser-4-amidino benzylamide,
benzylsulfonyl-D-hTyr-Ser-4-amidino benzylamide,
4-NH_{Z}-benzylsulfonyl-D-hPhe-Gly-4-amidino benzylamide,
benzylsulfonyl-D-indanylglycyl-Gly-4-amidino benzylamide,
benzylsulfonyl-D/L-hAla(4-pyridyl)-Gly-4-amidino benzylamide and
4-pyridylmethylsulfonyl-D-hPhe-Gly-4-amidino benzylamide,
wherein the C terminal aromatic benzamidine radical may be additionally substituted at 1, 2 or 3 positions with a halogen, in particular fluorine or chlorine, or with a methyl, ethyl, propyl, methoxy, ethoxy, or propoxy radical.

2. The compound according to claim 1, wherein at least one carboxyl group is in protected form as ester, preferably as ethyl ester.

3. The compound according to claim 1 or 2 in the form of a prodrug in which one or more carboxyl groups are present in the form of their alkyl esters with a branched or unbranched alkyl having 1-5 C atoms, preferably ethyl, and/or in which one or more hydroxyl groups are present in the form of carbonates in which the terminal radical is equal to R₇ as defined above, and/or in which the amidino- or guanidinobenzylamine radical is present in the form of hydroxyamidine or hydroxyguanidine, respectively, or of alkyloxycarbonyl derivative.

4. A compound of the general formula I 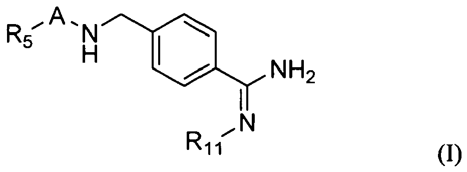 wherein
A is P₂—P₁ with
P₁ = 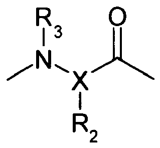 and
P₂ = 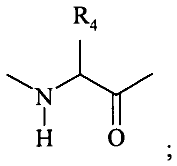
R₂ is an H, -CH₂-CH₂-COOH, -CH₂-CH₂-COOCH₂CH₃ or -CH₂OH;
R₃ is an H;
R₄ is a -(CH₂)₂-R₈, -CH₂NHR₈, -(CH₂)₂NHR₈ or a -CH₂-4-hydroxycyclohexyl radical, wherein R₈ is a mono- or polysubstituted or unsubstituted aryl or heteroaryl radical, wherein the aryl or heteroaryl radical has 5 or 6 C atoms and, in the case of a heteroaryl radical, 1 or 2 N as heteroatoms, and R₈ is preferably a phenyl, hydroxyphenyl, pyridyl or aminopyridyl radical;
R₅ is a methylsulfonyl, ethylsulfonyl, n-propylsulfonyl, isopropylsulfonyl, butylsulfonyl, n-butylsulfonyl, benzylsulfonyl, aminobenzylsulfonyl, hydroxybenzylsulfonyl, chlorobenzylsulfonyl, fluorobenzylsulfonyl, carboxybenzylsulfonyl, ethyloxycarbonylbenzylsulfonyl, carboxymethylbenzylsulfonyl, ethyloxycarbonylmethylbenzylsulfonyl, pyridylmethylenesulfonyl, N-(oxide)-pyridylmethylsulfonyl, -CH₂-COOH or a -CH₂COOCH₂CH₃ radical;
X is CH;
R₁₁ is H, OH or -COOR₁₂ with R₁₂ is a branched or unbranched alkyl radical with 2, 4 or 6 C atoms,
or a compound of the general formula I in the form of its salt;
with the exception of the following compounds:
benzylsulfonyl-D-hPhe-Ser-4-amidino benzylamide,
benzylsulfonyl-D-hAla(4-pyridyl)-Ser-4-amidino benzylamide,
benzylsulfonyl-D-hTyr-Ser-4-amidino benzylamide,
4-NH₂-benzylsulfonyl-D-hPhe-Gly-4-amidino benzylamide,
benzylsulfonyl-D-indanylglycyl-Gly-4-amidino benzylamide,
benzylsulfonyl-D/L-hAla(4-pyridyl)-Gly-4-amidino benzylamide and
4-pyridylmethylsulfonyl-D-hPhe-Gly-4-amidino benzylamide.

5. The compound according to at least one of claims 1 to 4, **characterized in that** R₄ is a -CH₂-CH₂-R₈ radical, wherein R₈ is an aryl or heteroaryl radical having 4-6 ring atoms, which has 1 or 2 heteroatoms, preferably N, and may be substituted by one or more -NH₂ and/or -OH groups, and preferably P₂ in the structure A of the general formula I is derived from a homophenylalanine, homotyrosine, indanylglycine or 4-pyridylhomoalanine, and the P₂ amino acid is present in particular in the D configuration.

6. The compound according to at least one of claims 1 to 5, **characterized in that** the substituent is -OH, -NH₂, -NO₂, -COOH, -COOCH₂CH₃, fluorine, chlorine or bromine, in particular fluorine or chlorine.

7. The compound according to at least one of claims 1 to 6, **characterized in that** the compounds are present in the form of salts, preferably with mineral acids or with suitable organic acids, preferably with hydrochloric acid, sulfuric acid, acetic acid, formic acid, methyl sulfonic acid, succinic acid, malic acid or trifluoroacetic acid, especially in the form of their hydrochlorides, sulfates or acetates.

8. A method for preparing a compound according to at least one of claims 1 to 7, **characterized in that** the appropriate amino acids are coupled sequentially onto an amidinobenzylamine which is protected on the amidino group, wherein the N-terminal amino acid either already carrying the R₅ radical or the latter subsequently being linked thereto.

9. A medicament comprising a compound according to at least one of claims 1 to 7 and pharmaceutically suitable excipients and/or additives.

10. The medicament according to claim 9, wherein the medicament is employed in the form of a tablet, of a coated tablet, of a capsule, of a pellet, suppository, of a solution, in particular of an injection or infusion solution, of eye drops, nose and ear drops, of a syrup, of an emulsion or suspension, of globules, styli, aerosol, powder, of a paste, cream or ointment.

11. Use of a compound according to at least one of claims 1 to 7 for the preparation of a medicament as Xa inhibitor for the therapy or prophylaxis of a cardiovascular disorder or of a thromboembolic event, in particular in oral, subcutaneous, intravenous or transdermal form.

12. A compound of the general formula I 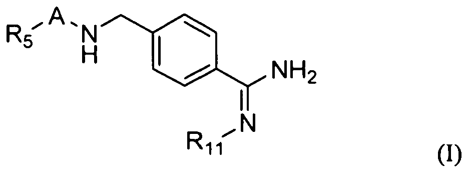 wherein
A is P₂—P₁ with
P₁ = 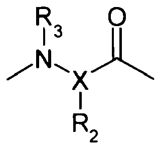 and
P₂ = 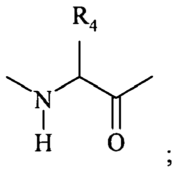
X = N or a CH, preferably CH;
R₂ is an -(CH₂)ₐCONHR₇* or -(CH₂)ₐCONHR₇** group, wherein a = 1, 2 or 3 and
R₇* is an H or a branched or unbranched alkyl radical 1-5 C atoms, preferably ethyl;
R₇** is an aryl radical or a heteroaryl radical with 1-2 heteroatoms selected from N, S or O;
R₃ is an H;
R₄ is -(CH₂)_{f}-R₈ with f = 0 or 2, preferably with f = 2, -CH₂NHR₈, -(CH₂)₂NHR₈ or -CH=CH-R₈, wherein R₈ is a mono- or polysubstituted or unsubstituted aryl or heteroaryl radical, wherein the aryl or heteroaryl radical preferably has 5 to 14, in particular 5 to 6 C atoms in the ring and, in the case of the heteroaryl radical, preferably possesses 1 to 3 N as heteroatoms, and wherein P₂ in the structure A of the general formula I is in the D or L configuration, preferably in the D configuration;
R₅ is -(CH₂)ᵢ-COOR₉ with i = 1, 2 or 3, preferably with i = 1, and R₉ is equal to a branched or unbranched alkyl radical having 1-5 C atoms, preferably ethyl, or R₅ is -SO₂R₉*, -SO₂-NH-R₉*, wherein R₉* is an H, a branched or unbranched alkyl having 1-10, preferably 1 to 6, in particular 1 to 4, especially 1 to 2 C atoms, a mono- or polysubstituted or unsubstituted aryl, heteroaryl, aralkyl, preferably benzyl, heteroaralkyl radical or a cyclohexylalkyl radical, preferably a cyclohexylmethyl radical, wherein the substituent may be an -OH, -O-COOR₇, -CH₂-OCOOR₇, with R₇ as defined above, -NH₂, -NO₂, -COOR₁₀, -CH₂-COOR₁₀ group or a Cl, F or Br atom, and wherein R₁₀ is an H or an alkyl radical having 1 to 6, in particular having 1 to 4 C atoms, especially ethyl;
R₁₁ is H, OH, or -COOR₁₂, wherein R₁₂ is a branched or unbranched alkyl radical having 1 to 8, preferably 1 to 6 c atoms;
or a compound of the general formula I in the form of its salt.

13. The compound according to claim 12, wherein in R₅ R₉ = H.

14. The compound according to claim 12 or 13, wherein R4 is a -CH₂-SR₈ or - CH₂CH₂-SR₈ group.

15. A compound selected from the following structures:

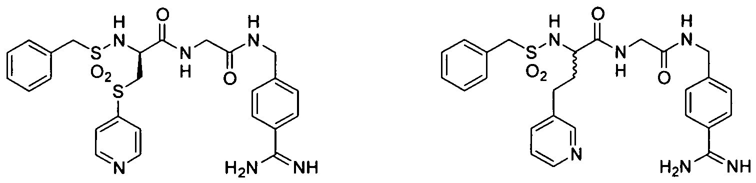

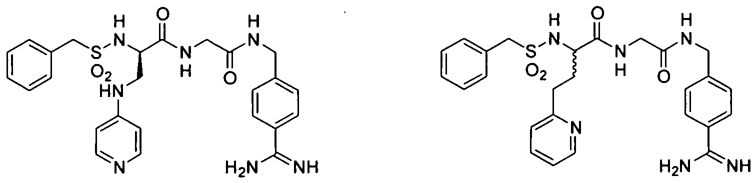

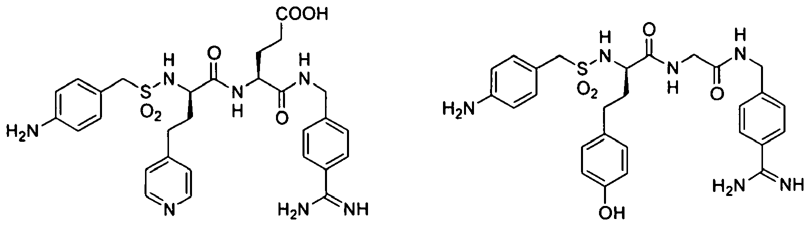

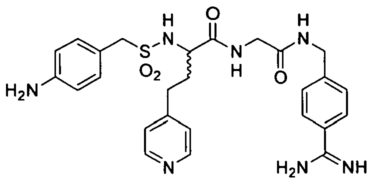

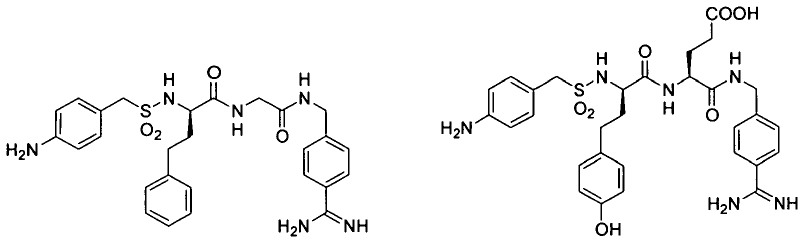

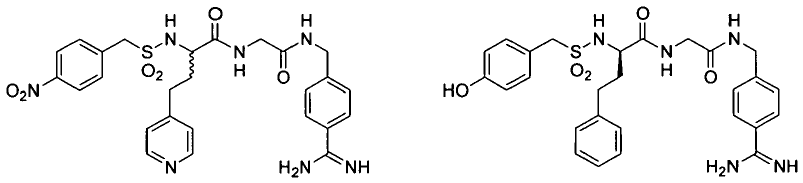

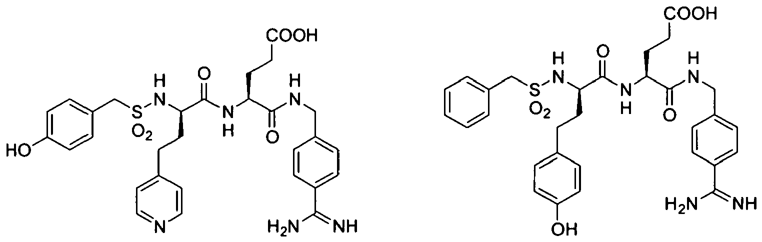

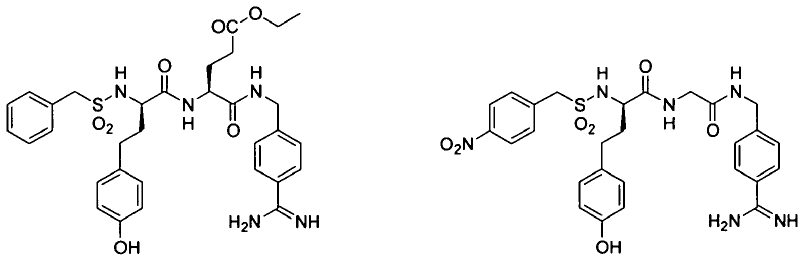

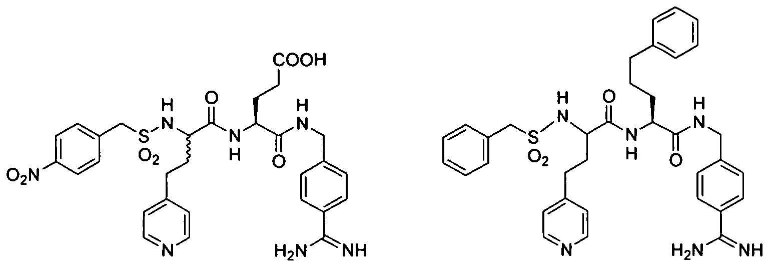

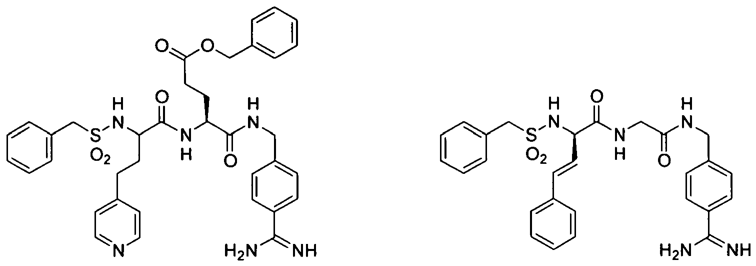

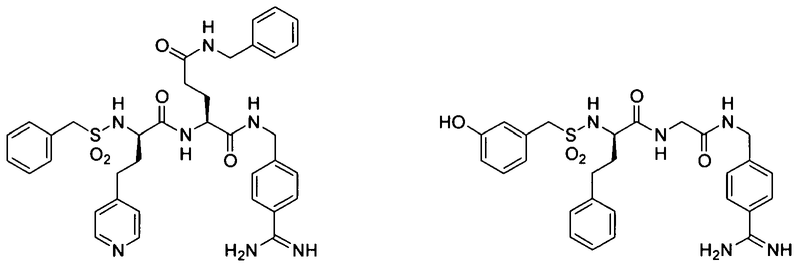

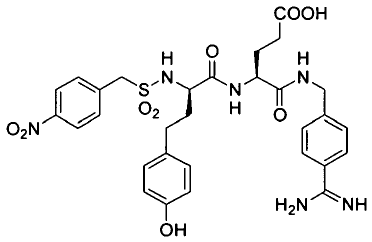

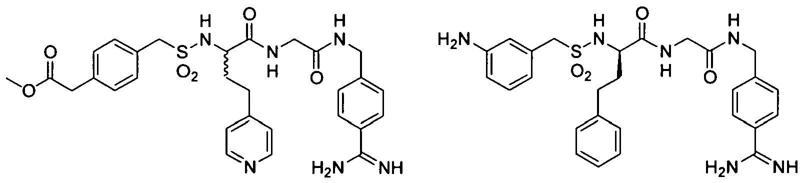

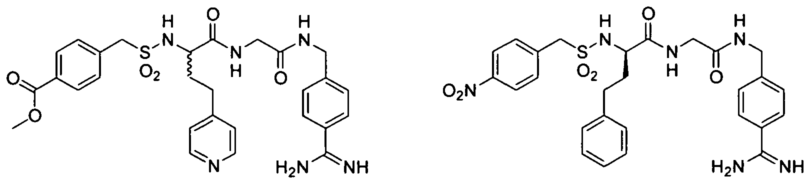

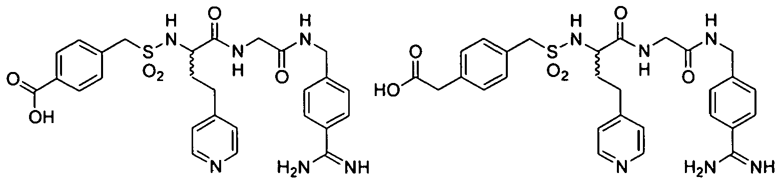

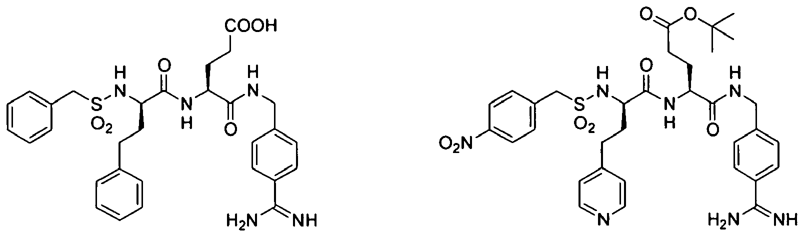

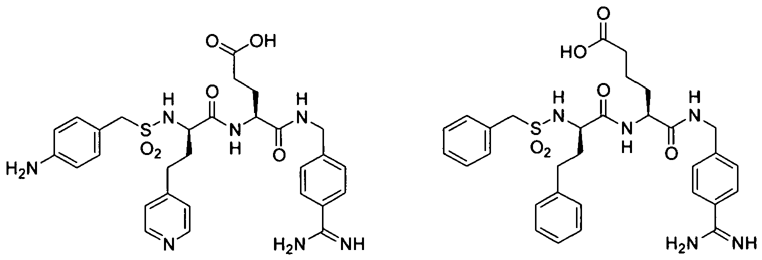

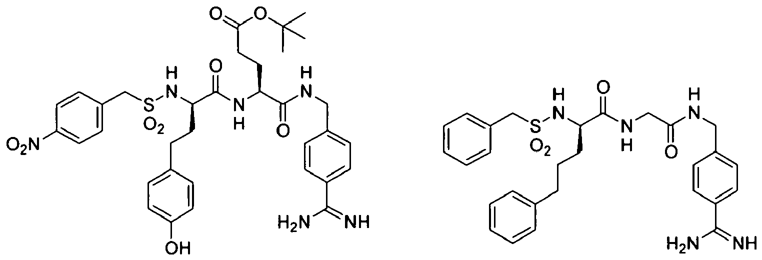

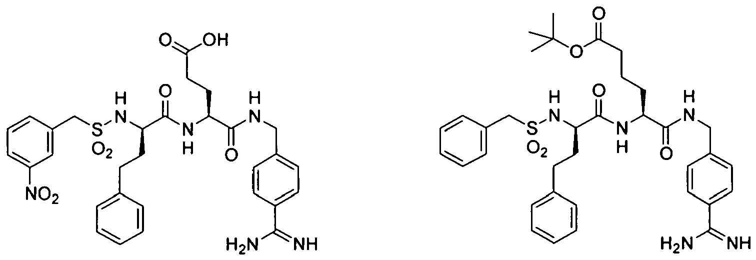

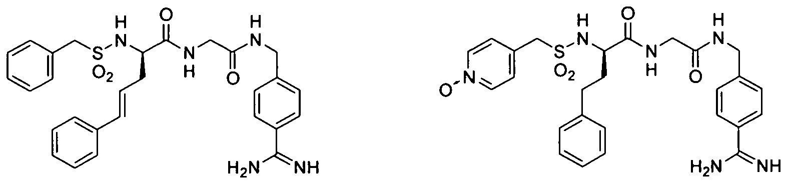

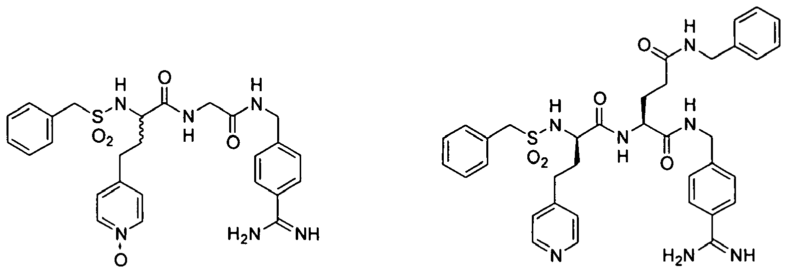

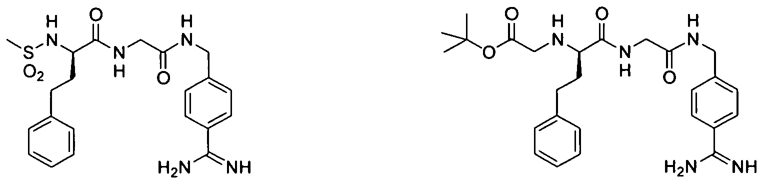

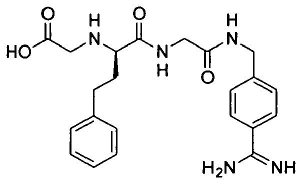

16. The compound according to at least one of claims 12 to 15, **characterized in that** the compounds are present in the form of salts, preferably with mineral acids or with suitable organic acids, preferably with hydrochloric acid, sulfuric acid, acetic acid, formic acid, methyl sulfonic acid, succinic acid, malic acid or trifluoroacetic acid, especially in the form of their hydrochlorides, sulfates or acetates.

17. A method for preparing a compound according to at least one of claims 12 to 16, **characterized in that** the appropriate amino acids are coupled sequentially onto an amidinobenzylamine which is protected on the amidino group, wherein the N-terminal amino acid either already carries the R₅ radical or the latter is subsequently linked thereto.

18. A medicament comprising a compound according to at least one of claims 12 to 16 and pharmaceutically suitable excipients and/or additives.

19. The medicament according to claim 18, wherein the medicament is employed in the form of a tablet, of a coated tablet, of a capsule, of a pellet, suppository, of a solution, in particular of an injection of infusion solution, of eye drops, nose and ear drops, of a syrup, of an emulsion or suspension, of globules, styli, aerosol, powder, of a paste, cream or ointment.

20. Use of a compound according to at least one of claims 12 to 16 for the preparation of a medicament as Xa inhibitor for the therapy or prophylaxis of a cardiovascular disorder or of a thromboembolic event, in particular in oral, subcutaneous, intravenous or transdermal form.

## Revendications

1. Composé de formule générale 1 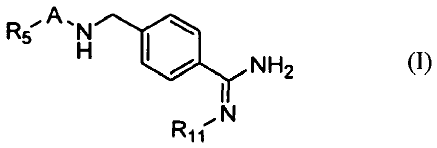 dans laquelle
A est P₂—P₁, avec 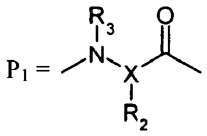 et 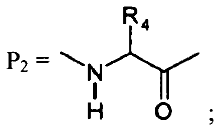 X est N ou CH, de préférence CH ;
R₂ est H, -CH₂-OR₇ ou -CH₂-OCOOR₇, où R₇ est H ou un groupe alkyle ramifié ou non ramifié contenant 1 à 5, en particulier de 1 à 3 atomes de carbone, ou bien R₂ est -CH₂-CH₂-COOR₇*, où R₇* est H ou un groupe alkyle ramifié ou non ramifié contenant 1 à 5 atomes de carbone, de préférence l'éthyle ;
R₃ est H ;
R₄ est -(CH₂)_{f}-R₈, avec f = 0 ou 2, de préférence f = 2, -CH₂NHR₈, -(CH₂)₂NHR₈ ou -CH=CH-R₈, où R₈ est un groupe aryle ou hétéroaryle mono- ou polysubstitué ou non substitué, lequel groupe aryle ou hétéroaryle contient de préférence 5 à 14, en particulier 5 à 6 atomes de carbone dans le noyau cyclique et, dans le cas du groupe hétéroaryle, possède de préférence 1 à 3 atomes d'azote comme hétéroatomes, et dans laquelle P₂ dans la structure A de la formule générale 1 est présent en configuration D ou L, de préférence en configuration D ;
R₅ est -(CH₂)ᵢ-COOR₉, avec i = 1; 2 ou 3, de préférence i = 1, et R₉ est un groupe alkyle ramifié ou non ramifié contenant 1 à 5 atomes de carbone, de préférence l'éthyle, ou bien R₅ est -SO₂R₉* -SO₂-NH-R₉* où R9* est H, un alkyle ramifié ou non ramifié contenant 1 à 10, de préférence 1 à 6, en particulier 1 à 4 et surtout 1 à 2 atomes de carbone, un groupe aryle, hétéroaryle, aralkyle, de préférence benzyle, un groupe hétéroaralkyle ou un groupe cyclohexylalkyle, de préférence un groupe cyclohexylméthyle, mono- ou polysubstitué ou non substitué, le substituant étant un groupe -OH, -O-COOR₇, -CH₂-OCOOR₇, où R₇ est tel que défini ci-dessus, -NH₂, -NO₂, -COOR₁₀, -CH₂-COOR₁₀ ou un atome de Cl, F ou Br, et R₁₀ est H ou un groupe alkyle contenant 1 à 6, en particulier 1 à 4 atomes de carbone, en particulier l'éthyle ;
R₁₁, est H, OH ou -COOR₁₂, où R₁₂ est un groupe alkyle ramifié ou non ramifié contenant 1 à 8, de préférence 1 à 6 atomes de carbone ;
ou un composé de formule générale 1 sous la forme de son sel ;
à l'exception des composés suivants :
benzylsulfonyl-D-hPhe-Ser-4-amidinobenzylamide,
benzylsulfonyl-D-hAla(4-pyridyl)-Ser-4-amidinobenzylamide,
benzylsulfonyl-D-hTyr-Ser-4-amidinobenzylamide,
4-NH₂-benzylsulfonyl-D-hPhe-Gly-4-amidinobenzylamide,
benzylsulfonyl-D-indanylglycyl-Gly-4-amidinobenzylamide,
benzylsulfonyl-D/L-hAla(4-pyridyl)-Gly-4-amidinobenzylamide et
4-pyridylméthylsulfonyl-D-hPhe-Gly-4-amidinobenzylamide,
dans laquelle le groupe benzamidine aromatique C-terminal peut être substitué en plus dans 1, 2 ou 3 positions par un halogène, en particulier le fluor ou le chlore, ou par un groupe méthyle, éthyle, propyle, méthoxy, éthoxy ou propoxy.

2. Composé selon la revendication 1, dans laquelle au moins un groupe carboxyle est présent protégé sous forme d'ester, de préférence sous forme d'ester éthylique.

3. Composé selon la revendication 1 ou la revendication 2 sous la forme d'un promédicament dans lequel un ou plusieurs groupes carboxyle sont présents sous la forme de leurs esters alkyliques avec un alkyle ramifié ou non ramifié contenant 1 à 5 atomes de carbone, de préférence l'éthyle, et/ou dans lequel un ou plusieurs groupes hydroxyle sont présents sous la forme de carbonates dans lesquels le groupe terminal est égal à R₇ tel que défini ci-dessus, et/ou dans lequel le groupe amidino- ou guanidinobenzylamine est présent sous la forme d'hydroxyamidine ou d'hydroxyguanidine ou sous la forme d'un dérivé alkyloxycarbonyle.

4. Composé de formule générale 1 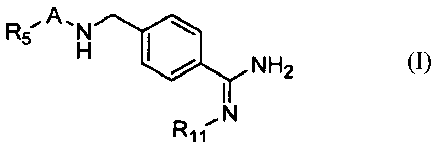 dans laquelle
A est P₂—P₁, avec 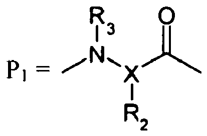 Et 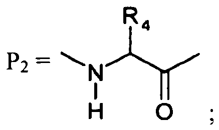 R₂ est H, -CH₂-CH₂-COOH, -CH₂-CH₂-COOCH₂CH₃ ou -CH₂OH ;
R₃ est H ;
R₄ est -(CH₂)₂-R₈, -CH₂NHR₈, -(CH₂)₂NHR₈ ou un groupe -CH₂-4-hydroxycyclohexyle, où R₈ est un groupe aryle ou hétéroaryle mono- ou polysubstitué ou non substitué, lequel groupe aryle ou hétéroaryle contient 5 ou 6 atomes de carbone et, dans le cas d'un groupe hétéroaryle, possède 1 ou 2 atomes d'azote comme hétéroatomes, de préférence R₈ est un groupe phényle, hydroxyphényle, pyridyle ou aminopyridyle ;
R₅ est un groupe méthylsulfonyle, éthylsulfonyle, n-propylsulfonyle, isopropyl-sulfonyle, butylsulfonyle, n-butylsulfonyle, benzylsulfonyle, aminobenzylsulfonyle, hydroxybenzylsulfonyle, chlorobenzylsulfonyle, fluorobenzylsulfonyle, carboxy-benzylsulfonyle, éthyloxycarbonylbenzylsulfonyle, carboxyméthylbenzylsulfonyle, éthyloxycarbonylméthylbenzylsulfonyle, pyridylméthylènesulfonyle, N-(oxyde)-pyridylméthylènesulfonyle, -CH₂-COOH ou -CH₂COOCH₂CH₃ ;
X est CH ;
R₁₁ est H, OH ou -COOR₁₂, où R₁₂ est un groupe alkyle ramifié ou non ramifié contenant 2, 4 ou 6 atomes de carbone,
ou un composé de formule générale I sous la forme de son sel ;
à l'exception des composés suivants:
benzylsulfonyl-D-hPhe-Ser-4-amidinobenzylamide,
benzylsulfonyl-D-hAla(4-pyridyl)-Ser-4-amidinobenzylamide,
benzylsulfonyl -D-hTyr- Ser-4-amidinobenzylamide,
4-NH₂-benzylsulfonyl-D-hPhe-Gly-4-amidinobenzylamide,
benzylsulfonyl-D-indanylglycyl-Gly-4-amidinobenzylamide,
benzylsulfonyl-D/L-hAla(4-pyridyl)-G y-4-amidinobenzylamide et
4-pyridylméthylsulfonyl-D-hPhe-Gly-4-amidinobenzylamide.

5. Composé selon l'une au moins des revendications 1 à 4, **caractérisé en ce que** R₄ est un groupe -CH₂-CH₂-R₈, où R₈ est un groupe aryle ou hétéroaryle qui contient 4 à 6 atomes dans le noyau cyclique, qui possède 1 ou 2 hétéroatomes, de préférence N, et qui est substitué par un ou plusieurs groupes -NH₂ et/ou -OH, de préférence P₂ dans la structure A de la formule générale I est dérivé d'une homophénylalanine, d'une homotyrosine, d'une indanylglycine ou d'une 4-pyridylhomoalanine et l'acide aminé P₂ est présent en particulier en configuration D.

6. Composé selon l'une au moins des revendications 1 à 5; **caractérisé en ce que** le substituant est un groupe -OH, -NH₂, -NO₂, -COOH, -COOCH₂CH₃ ou un atome de fluor, de chlore ou de brome, en particulier de fluor ou de chlore.

7. Composé selon l'une au moins des revendications 1 à 6, **caractérisé en ce que** les composés sont présents sous forme de sels, de préférence avec des acides minéraux ou avec des acides organiques appropriés, de préférence avec l'acide chlorhydrique, l'acide sulfurique, l'acide acétique, l'acide formique, l'acide méthanesulfonique, l'acide succinique, l'acide malique ou l'acide trifluoroacétique et surtout sous la forme de leur chlorhydrates, leurs sulfates ou leurs acétates.

8. Procédé de préparation d'un composé selon l'une au moins des revendications 1 à 7, **caractérisé en ce que** les acides aminés correspondants sont couplés séquentiellement à une amidinobenzylamine protégée au niveau du groupe amidino, l'acide aminé N-terminal portant déjà le groupe R₅ ou bien étant lié à celui-ci par la suite.

9. Médicament contenant un composé selon l'une au moins des revendications 1 à 7 ainsi que des adjuvants et/ou des additifs pharmaceutiquement acceptables.

10. Médicament selon la revendication 9, ledit médicament étant utilisé sous la forme d'un comprimé, d'une dragée, d'une gélule, d'un granulé, d'un suppositoire, d'une solution, en particulier d'une solution injectable ou perfusable, de gouttes ophtalmiques, nasales et auriculaires, d'un sirop, d'une émulsion ou suspension, d'un globule, d'un stylet, d'un aérosol, d'une poudre, d'une pâte, d'une crème ou d'une pommade.

11. Utilisation d'un composé selon l'une au moins des revendications 1 à 7 pour fabriquer un médicament agissant comme un inhibiteur du facteur Xa pour le traitement ou la prévention d'une maladie cardiovasculaire ou d'un épisode thrombo-embolique, en particulier par voie orale, sous-cutanée, intraveineuse ou percutanée.

12. Composé de formule générale I 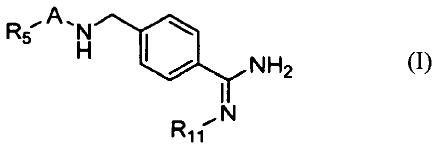 dans laquelle
A est P₂—P₁, avec 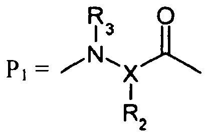 et 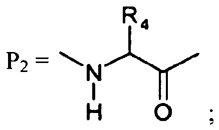 X est N ou CH, de préférence CH ;
R₂ est un groupe -(CH₂)ₐCONHR₇* ou -(CH₂)aCONHR₇**, où a = 1, 2 ou 3 et
R₇* est H ou un groupe alkyle ramifié ou non ramifié contenant 1 à 5 atomes de carbone, de préférence l'éthyle ;
R₇** est un groupe aryle ou un groupe hétéroaryle contenant 1 ou 2 hétéroatomes choisis parmi N, S ou O :
R³ est H ;
R₄ est -(CH₂)_{f}R₈, avec f = 0 ou 2, de préférence f = 2, -CH₂NHR₈, -(CH₂)₂NHR₈ ou -CH=CH-R₈, où R₈ est un groupe aryle ou hétéroaryle mono- ou polysubstitué ou non substitué, lequel groupe aryle ou hétéroaryle contient 5 à 14, en particulier 5 à 6 atomes de carbone dans le noyau cyclique et, dans le cas du groupe hétéroaryle, possède de préférence 1 à 3 atomes d'azote comme hétéroatomes, et dans laquelle P₂ dans la structure A de la formule générale 1 est présent en configuration D ou L, de préférence en configuration D ;
R₅ est -(CH₂)ᵢ-COOR₉, avec i = 1, 2 ou 3, de préférence i = 1, et R₉ est un groupe alkyle ramifié ou non ramifié contenant 1 à 5 atomes de carbone, de préférence l'éthyle, ou bien R₅ est -SO₂R₉*, -SO₂-NH-R₉*, où R₉* est H, un groupe alkyle ramifié ou non ramifié contenant 1 à 10, de préférence 1 à 6, en particulier 1 à 4 et surtout 1 à 2 atomes de carbone, un groupe aryle, hétéroaryle, aralkyle, de préférence un groupe benzyle, un groupe hétéroaralkyle ou un groupe cyclohexylalkyle mono- ou polysubstitué ou non substitué, de préférence un groupe cyclohexylméthyle, le substituant étant un groupe - OH, -O-COOR₇, -CH₂-OCOOR₇, où R₇ est tel que défini ci-dessus, -NH₂, -NO₂, - COOR₁₀, -CH₂-COOR₁₀ ou un atome de Cl, F ou Br, et R₁₀ est H ou un groupe alkyle contenant 1 à 6, en particulier 1 à 4 atomes de carbone et surtout l'éthyle ;
R₁₁, est H, OH ou -COOR₁₂, où R₁₂ est un groupe alkyle ramifié ou non ramifié contenant 1 à 8, de préférence 1 à 6 atomes de carbone ;
ou un composé de formule générale I sous la forme de son sel.

13. Composé selon la revendication 12, dans laquelle, dans R₅, R₉ est H.

14. Composé selon la revendication 12 ou la revendication 13, où R₄ est un groupe -CH₂-SR₈ ou -CH₂CH₂SR₈.

15. Composé choisi parmi les structures suivantes :

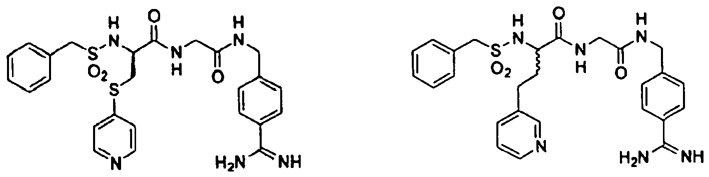

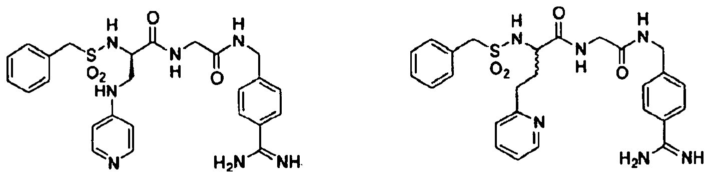

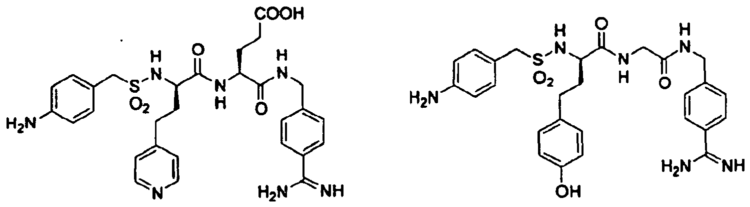

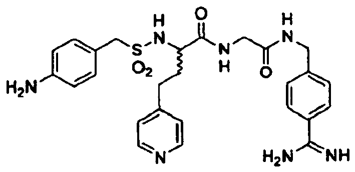

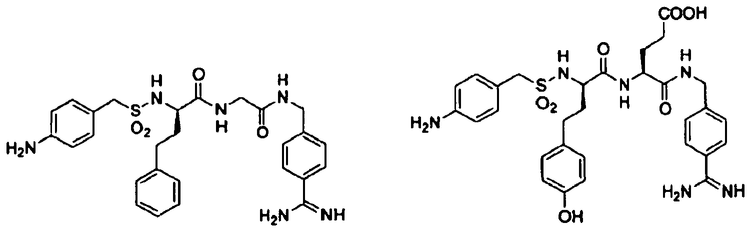

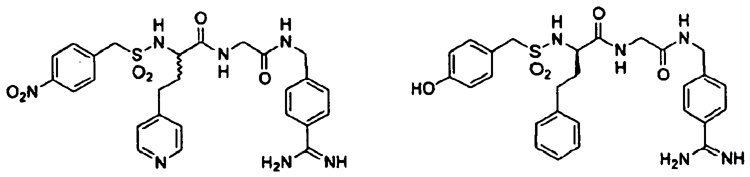

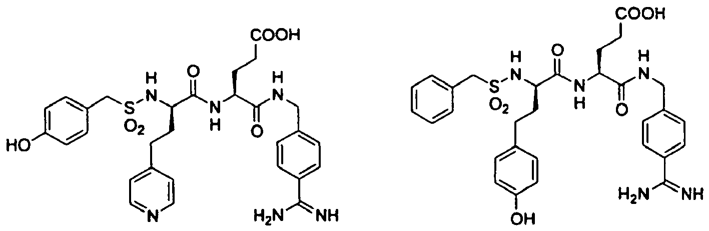

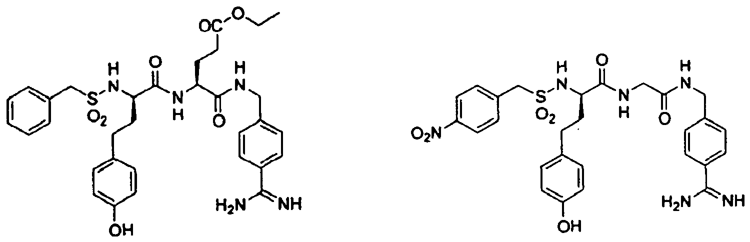

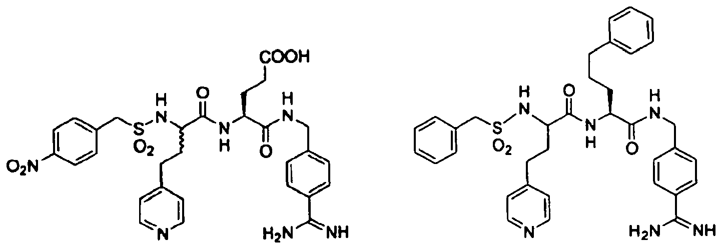

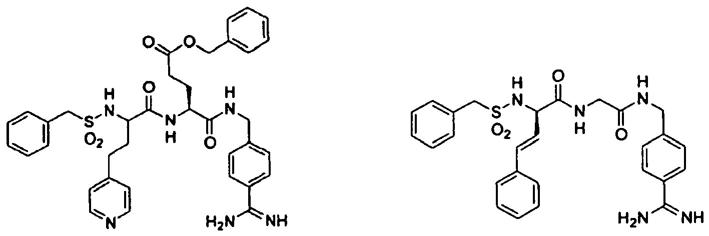

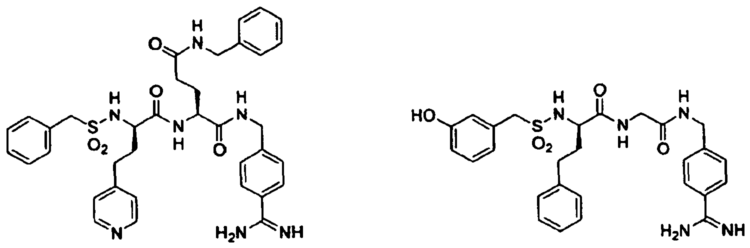

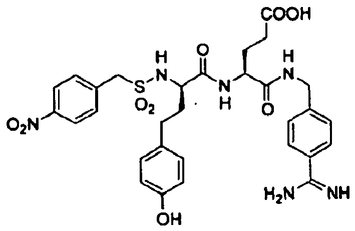

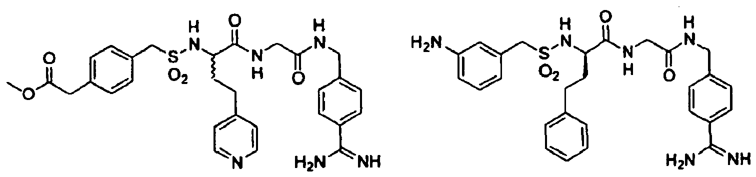

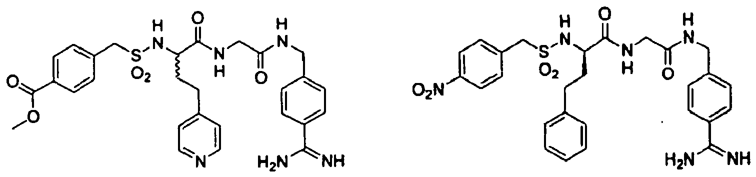

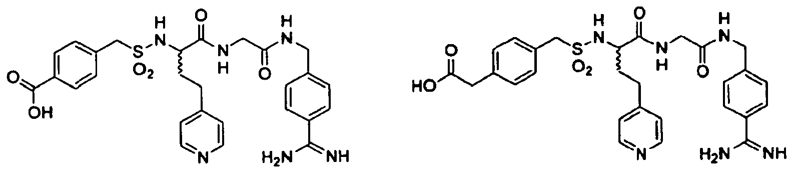

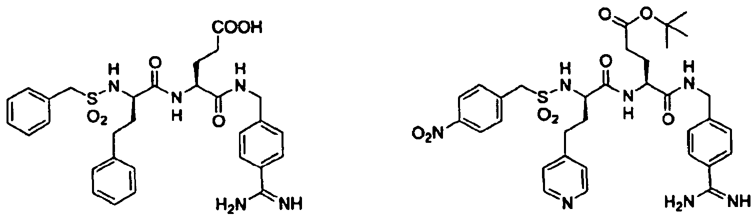

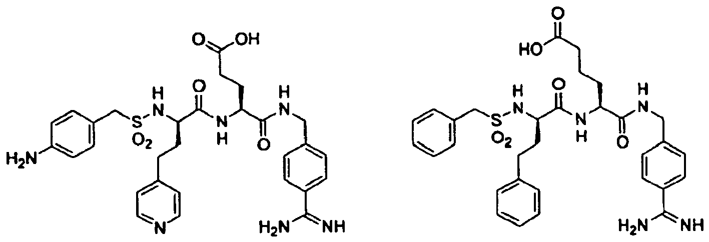

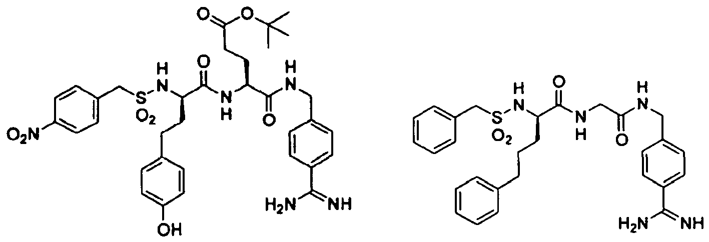

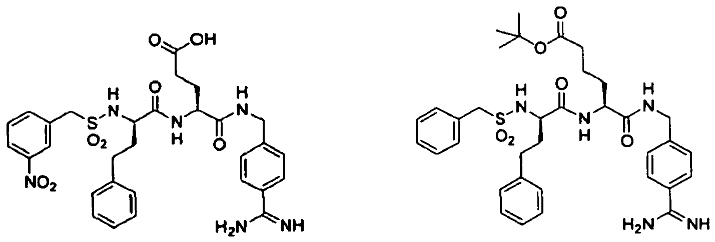

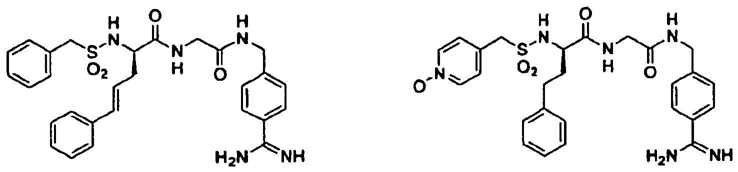

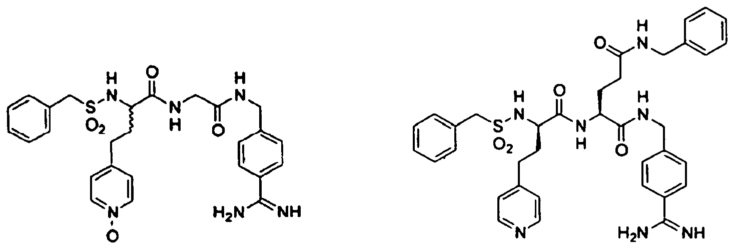

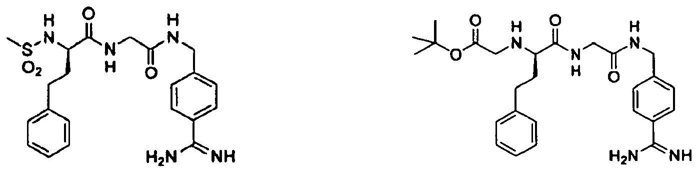

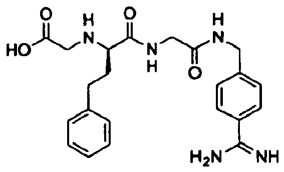

16. Composé selon l'une au moins des revendications 12 à 15, **caractérisé en ce que** les composés sont présents sous forme de sels, de préférence avec des acides minéraux ou avec des acides organiques appropriés, de préférence avec l'acide chlorhydrique, l'acide sulfurique, l'acide acétique, l'acide formique, l'acide méthanesulfonique, l'acide succinique, l'acide malique ou l'acide trifluoroacétique et surtout sous la forme de leurs chlorhydrates, leurs sulfates ou leurs acétates.

17. Procédé de préparation d'un composé selon l'une au moins des revendications 12 à 16, **caractérisé en ce que** les acides aminés correspondants sont couplés séquentiellement à une amidinobenzylamine protégée au niveau du groupe amidino, l'acide aminé N-terminal portant déjà le groupe R₅ ou bien étant lié à celui-ci par la suite.

18. Médicament contenant un composé selon l'une au moins des revendications 12 à 16 ainsi que des adjuvants et/ou des additifs pharmaceutiquement acceptables.

19. Médicament selon la revendication 18, dans laquelle le médicament est utilisé sous la forme d'un comprimé, d'une dragée, d'une gélule, d'un granulé, d'un suppositoire, d'une solution, en particulier d'une solution injectable ou perfusable, de gouttes ophtalmiques, nasales et auriculaires, d'un sirop, d'une émulsion ou suspension, d'un globule, d'un stylet, d'un aérosol, d'une poudre, d'une pâte, d'une crème ou d'une pommade.

20. Utilisation d'un composé selon l'une au moins des revendications 12 à 16 pour fabriquer un médicament agissant comme un inhibiteur du facteur Xa pour le traitement ou la prévention d'une maladie cardiovasculaire ou d'un épisode thrombo-embolique, en particulier par voie orale, sous-cutanée, intraveineuse ou percutanée.
